(19) Europäisches Patentamt
European Patent Office
Office européen des brevets

(11) EP 3 538 534 B1

(12) EUROPÄISCHE PATENTSCHRIFT

(45) Veröffentlichungstag und Bekanntmachung des Hinweises auf die Patenterteilung:
**16.09.2020 Patentblatt 2020/38**

(21) Anmeldenummer: **17804820.3**

(22) Anmeldetag: **07.11.2017**

(51) Int Cl.:
***C07F 5/02*** *(2006.01)* ***C08F 2/50*** *(2006.01)*
***G03F 7/029*** *(2006.01)*

(86) Internationale Anmeldenummer:
**PCT/EP2017/078412**

(87) Internationale Veröffentlichungsnummer:
**WO 2018/087062 (17.05.2018 Gazette 2018/20)**

(54) **VERFAHREN ZUR HERSTELLUNG VON TRIARYL-ORGANOBORATEN**

PROCESS FOR THE MANUFACTURING OF TRIARYL-ALKYL BORATES

PROCÉDÉ DE FABRICATION DE TRIARYL-ALKYLBORATES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.11.2016 EP 16197993**
**22.09.2017 EP 17192517**

(43) Veröffentlichungstag der Anmeldung:
**18.09.2019 Patentblatt 2019/38**

(73) Patentinhaber: **Covestro Intellectual Property GmbH & Co. KG**
**51373 Leverkusen (DE)**

(72) Erfinder:
- **RÖLLE, Thomas**
  **51381 Leverkusen (DE)**
- **BERNETH, Horst**
  **51373 Leverkusen (DE)**
- **HÖNEL, Dennis**
  **53909 Zülpich-Wichterich (DE)**
- **BRUDER, Friedrich-Karl**
  **47802 Krefeld (DE)**

(74) Vertreter: **Levpat**
**c/o Covestro AG**
**Gebäude 4825**
**51365 Leverkusen (DE)**

(56) Entgegenhaltungen:
**WO-A1-2014/053408 WO-A1-2015/187766**
**GB-A- 2 307 474 JP-A- 2002 226 486**
**JP-A- 2012 211 938**

- **YASUMASA TOBA: "Design of Photoinitiator System with Onium Berates.", KOBUNSHI RONBUNSHU (JAPANESE POLYMER SCIENCE AND TECHNOLOGY)., Bd. 59, Nr. 8, 1. Januar 2002 (2002-01-01) , Seiten 449-459, XP55458491, JP ISSN: 0386-2186, DOI: 10.1295/koron.59.449**
- **JANINA KABATC ET AL: "The application of halomethyl 1,3,5-triazine as a photoinitiator or co-initiator for acrylate monomer polymerization", JOURNAL OF PHOTOCHEMISTRY AND PHOTOBIOLOGY, A: CHEMISTRY, ELSEVIER SEQUOIA, LAUSANNE, CH, Bd. 219, Nr. 1, 20. Januar 2011 (2011-01-20), Seiten 16-25, XP028368558, ISSN: 1010-6030, DOI: 10.1016/J.JPHOTOCHEM.2011.01.011 [gefunden am 2011-01-28]**
- **Allonas: "Borate salts as coinitiators in photoinitiating szstems for laser imaging", Journal of Photopolymer Science and Technology, 1. Januar 2011 (2011-01-01), Seiten 531-534, XP55458467, Gefunden im Internet: URL:https://www.jstage.jst.go.jp/article/photopolymer/24/5/24_5_531/_pdf/-char/en [gefunden am 2018-03-12]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 538 534 B1

## Beschreibung

Die Erfindung betrifft ein Verfahren zur Herstellung von Triaryl-organoboraten ausgehend von Organo-Boronsäureestern in Gegenwart eines n-wertigen Kations 1/n $K^{n+}$.

Triaryl-organoborate können zusammen mit geeigneten Sensibilisatoren wie beispielsweise kationischen, anionischen oder neutralen Farbstoffen Typ(II) Photoinitiatoren bilden, die durch aktinische Strahlung eine radikalische Photopolymerisation geeigneter Monomere auslösen. Ihre Herstellung ist breit beschrieben und ausgewählte Tetraalkylammonium-triaryl-alkylborate sind kommerziell erhältlich.

DE 198 50 139 A1 beschreibt die Synthese von Tetraalkylammonium-triaryl-alkylboraten ausgehend von einem Alkyl- oder Cycloalkyl-Boronsäureester und deren Umsetzung mit drei Äquivalenten eines metallorganischen Reagenzes insbesondere einem Grignard-Reagenz. Dabei wird in einem ersten Schritt der Alkyl- oder Cycloalkyl-Boronsäureester hergestellt, isoliert und dieser Alkyl- oder Cycloalkyl-Boronsäureester anschließend mit einem separat hergestellten metallorganischen Reagenz, i.d.R. einem Grignard-Reagenz, umgesetzt. In einem dritten Schritt wird das so entstandene Triaryl-alkylborat durch Zugabe einer Tetraalkylammonium-Verbindung als Salz gefällt und z.B. durch Kristallisation gereinigt. Somit ergeben sich zur Herstellung von Tetraalkylammonium-triaryl-alkylboraten insgesamt 3 Synthese Stufen. Da die Löslichkeit von metallorganischen Reagenzien insbesondere Grignard-Reagenzien in den üblicherweise verwendeten ätherischen Lösungsmitteln begrenzt ist, muss bei diesem Verfahren insbesondere auf der Stufe des metallorganischen Reagenzes in recht verdünnter Lösung gearbeitet werden, um alle Inhaltsstoffe homogen in Lösung zu halten. Die Löslichkeit von Grignard-Reagenzien in den typischerweise verwendeten Ethern Tetrahydrofuran oder Diethylether beträgt üblicherweise 1 bis 2 Mol pro Liter. Da jedoch auf Grund der Stöchiometrie drei Äquivalente Grignard-Reagenz benötigt werden, liegt die Zielkonzentration an Produkt bei einem Drittel dieses Wertes, d.h. die Löslichkeit des Grignard-Reagenzes gibt die Zielkonzentration vor. Für technische Anwendung ist eine optimierte Raum-Zeit-Ausbeute ein wichtiges Ziel. Dies kann nach den in der DE 198 50 139 A1 beschriebenen Verfahren jedoch nur begrenzt erreicht werden.

JP2002-226486 A beschreibt die Synthese von Ammonium- und Phosphonium-triaryl-alkylboraten ausgehend von einem Alkyl- oder Cycloalkyl-Boronsäureester und deren Umsetzung mit drei Äquivalenten eines metallorganischen Reagenzes insbesondere einem Grignard-Reagenz nach einer *in situ* Methode (Barbier), bei der das metallorganische Reagenz in Gegenwart des elektrophilen Alkyl- oder Cycloalkyl-Boronsäureesters erzeugt wird. So können höhere Raum-Zeit-Ausbeuten erzielt werden und die Kontrolle der Exothermie der metallorganischen Stufe gelingt so besser. Darüber hinaus sind reaktive metallorganische Reagenzien teilweise nur bedingt lagerstabil und müssen mit hohem Aufwand vor Wasser oder Luft-Sauerstoff geschützt werden, da sie ansonsten hydrolysieren oder oxidiert werden, was wiederum die Gesamtausbeute reduziert.

Beide oben beschriebenen Verfahren erfordern jedoch noch die Zugabe des gewählten Kations in einem weiteren Reaktionsschritt. Für eine wirtschaftliche Herstellung im industriellen Maßstab ist es vorteilhaft, wenn das gewählte Kation bereits bei der Herstellung des Triaryl-organoborats vorliegt, weil so ein weiterer Reaktionsschritt gespart und die Durchführung und Aufarbeitung effizienter gestaltet werden kann. Zudem können die Konzentration der Reagenzien weiter erhöht werden, da die ansonsten anfallenden Metall-Aryl-Ammonium-Salze in den verwendeten Lösungsmittel nur begrenzt löslich sind und durch voluminös ausfallenden Niederschlag die Kontrolle der Reaktionsenthalpie verhindern.

GB 2 307 474 A offenbart Organobor-Photoinitiatoren.

Es war daher die Aufgabe der vorliegenden Erfindung, ein Verfahren zur Herstellung von Triaryl-organoboraten bereit zu stellen, bei dem eine verbesserte Raum-Zeit-Ausbeute zu einer besseren Wirtschaftlichkeit führt.

Diese Aufgabe wurde erfindungsgemäß durch ein Verfahren zur Herstellung von Triaryl-organoboraten der Formel $1/n\ K^{n+}R^4_3B^-\text{-}R^1$ (IV) gelöst, bei dem ein Äquivalent Organo-Boronsäureester der Formel $B\text{-}R^1(OR^2)(OR^3)$ (I) mit 1/n Äquivalenten Salz $K^{n+}\ nX^-$ (II) und 3 Äquivalenten Metall M in einem Lösungsmittel oder einem Lösungsmittelgemisch S vorgelegt, 3 Äquivalente eines Halogen-Aromaten $R^4$-Y (III) zugegeben werden, Wasser und gegebenenfalls ein zweites organisches Lösungsmittel S zugegeben und die Verbindung $1/n\ K^{n+}R^4_3B^-\text{-}R^1$ (IV) mit der organischen Phase abgetrennt wird,
worin

$R^1$ für einen gegebenenfalls mit Hydroxy und / oder Alkoxy und / oder Acyloxy und / oder Halogen substituierten $C_1$- bis $C_{22}$-Alkyl-, $C_3$- bis $C_{22}$-Alkenyl-, $C_3$- bis $C_{22}$-Alkinyl-, $C_5$- bis $C_7$-Cycloalkyl- oder $C_7$- bis $C_{15}$-Aralkylrest steht,

$R^2$ und $R^3$ unabhängig voneinander für einen gegebenenfalls verzweigten $C_1$- bis $C_{22}$-Alkyl-Rest oder einen gegebenenfalls durch Alkyl substituierten $C_3$- bis $C_7$-Cycloalkyl-Rest stehen oder $R^2$ und $R^3$ gemeinsam eine

2-8 gliedrige gegebenenfalls durch Alkyl substituierte und / oder durch Sauerstoff-Atome unterbrochene Kohlenstoff-Brücke bilden,

$R^4$ für einen $C_6$- bis $C_{14}$-Arylrest steht, der gegebenenfalls durch mindestens einen Rest der Auswahl Halogen, $C_1$-bis $C_4$-Alkyl, Trifluormethyl, $C_1$-bis $C_4$-Alkoxy, Trifluormethoxy, Phenyl und / oder Phenoxy substituiert ist,

K für ein beliebig substituiertes Organokation der Wertigkeit n auf Basis von Stickstoff, Phosphor, Sauerstoff, Schwefel und / oder Iod steht,

M für ein Metall beliebig ausgewählt aus den Alkalimetallen, Magnesium, Calcium oder Aluminium steht,

X für ein Halogenid oder Alkoxid oder Alkylsulfid steht,

Y für Iod oder Brom oder Chlor steht,

n für 1, 2 oder 3 steht und

S für ein aprotisches organisches Lösungsmittel oder ein Gemisch aprotischer organischer Lösungsmittel steht.

Dabei wird durch Zugabe von 3 Äquivalenten eines Halogen-Aromaten $R^4$-Y (III) zu den vorgelegten 3 Äquivalenten Metall *in situ* ein metallorganisches Reagenz erzeugt, das mit den vorgelegten Substanzen (I) und (II) zum Triaryl-organoborat $1/n\ K^{n+}R^4_3B^{-}\text{-}R^1$ (IV) reagiert. Überraschenderweise wurde gefunden, dass sich die Verwendung von substituierten Organokationen der Wertigkeit n auf Basis von Stickstoff, Phosphor, Sauerstoff, Schwefel und / oder Iod als Kationen vorteilhaft auf die Reaktion auswirkt, da sie chemisch inert sind und die Reaktion mit einer höheren Raum-Zeitausbeute als bekannte Verfahren verläuft und weniger Nebenreaktionen auftreten und damit die Ausbeute erhöht wird und eine höhere Reinheit des Zielproduktes sehr viel leichter zu erzielen ist.

Als weiterhin vorteilhaft erweist sich die Aufarbeitung durch Zugabe von Wasser und gegebenenfalls eines weiteren Lösungsmittels, da die gewünschten Triaryl-organoborate der Formel 1/n $K^{n+}R^4_3B^{-}\text{-}R^1$ (IV) in der organischen Phase verbleiben und die entstehenden Metallsalze $MY(OR^2)$, $MY(OR^3)$ und MXY mit der wässrigen Phase leicht abgetrennt werden können.

Die Triaryl-organoborate gemäß Formel IV sind vorzugsweise Triaryl-alkylborate, Triaryl-cycloalkylborate, Triaryl-alkenylborate, Triaryl-alkinylborate und / oder Triaryl-aralkylborate, bevorzugter Triaryl-alkylborate und / oder Triaryl-cycloalkylborate.

Ebenfalls Gegenstand der Erfindung ist daher ein Verfahren zur Herstellung von Verbindungen der Formel

$1/n\ K^{n+}R^4_3B^{-}\text{-}R^1$ (IV)

umfassend die Schritte

i) das Vorlegen von einem Äquivalent Organo-Boronsäureester $B\text{-}R^1(OR^2)(OR^3)$ (I) mit 1/n Äquivalenten Salz $K^{n+}$ $nX^-$ (II) und 3 Äquivalenten Metall M in einem Lösungsmittel oder einem Lösungsmittelgemisch S, und
ii) die Zugabe von 3 Äquivalenten eines Halogen-Aromaten $R^4$-Y (III), wodurch
iii) ein *in situ* erzeugtes metallorganisches Reagenz mit den vorgelegten Substanzen (I) und (II) zum $1/n\ K^{n+}R^4_3B^{-}\text{-}R^1$ (IV) reagiert.

Daran anschließen können sich die Schritte

iv) Zugabe von Wasser und gegebenenfalls eines weiteren Lösungsmittels, und

v) Abtrennen der gewünschten Triaryl-organoborate der Formel 1/n $K^{n+}R^4_3B^{-}\text{-}R^1$ $K^{n+}$ (IV) mit der organischen Phase.

Dabei genügt die Umsetzung der folgenden Gesamt-Reaktionsgleichung:

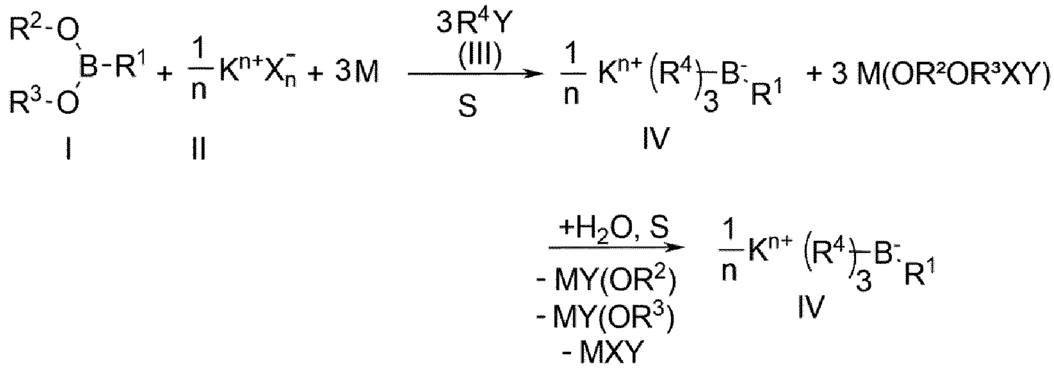

I II IV
IV

Bevorzugt steht $R^1$ für einen gegebenenfalls mit Hydroxy und / oder Alkoxy und / oder Acyloxy und / oder Halogen substituierten $C_2$- bis $C_{18}$-Alkyl-, $C_3$- bis $C_{18}$-Alkenyl-, $C_3$- bis $C_{18}$-Alkinyl-, $C_5$-bis $C_6$-Cycloalkyl- oder $C_7$- bis $C_{13}$-Aralkylrest und besonders bevorzugt steht $R^1$ für einen gegebenenfalls mit Hydroxy und / oder Alkoxy und / oder Acyloxy und / oder Halogen substituierten $C_4$-bis $C_{16}$-Alkyl-, $C_3$- bis $C_{16}$-Alkenyl-, $C_3$- bis $C_{16}$-Alkinyl-, Cyclohexyl- oder $C_7$- bis $C_{13}$-Aralkylrest.

Bevorzugt stehen $R^2$ und $R^3$ unabhängig voneinander für einen gegebenenfalls verzweigten $C_2$- bis $C_{18}$-Alkyl-Rest oder $R^2$ und $R^3$ bilden gemeinsam einen 4-7 gliedrigen gegebenenfalls substituierten Carbocylus und besonders bevorzugt stehen $R^2$ und $R^3$ unabhängig voneinander für einen gegebenenfalls verzweigten $C_3$- bis $C_{12}$-Alkyl-Rest oder $R^2$ und $R^3$ bilden gemeinsam einen 4-6 gliedrigen gegebenenfalls substituierten Carbocylus.

Bevorzugt steht $R^4$ für einen $C_6$- bis $C_{10}$-Arylrest, der gegebenenfalls durch mindestens einen Rest der Auswahl Halogen, $C_1$-bis $C_4$-Alkyl, Trifluormethyl, $C_1$-bis $C_4$-Alkoxy, Trifluormethoxy, Phenyl und / oder Phenoxy substituiert ist und besonders bevorzugt steht $R^4$ für einen $C_6$-Arylrest, der gegebenenfalls durch mindestens einen Rest der Auswahl Halogen, $C_1$-bis $C_4$-Alkyl, Trifluormethyl, $C_1$-bis $C_4$-Alkoxy, Trifluormethoxy, Phenyl und / oder Phenoxy substituiert ist.

Unter einem beliebig substituierten Organokation K der Wertigkeit n auf Basis von Stickstoff werden beispielsweise Ammoniumionen, Pyridiniumionen, Pyridaziniumionen, Pyrimidiniumionen, Pyraziniumionen, Imidazoliumionen, 1H-Pyrazoliumionen, 3H-Pyrazoliumionen, 4H-Pyrazoliumionen, 1-Pyrazoliniumionen, 2-Pyrazoliniumionen, 3-Pyrazoliniumionen, Imidazoliniumionen, Thiazoliumionen, 1,2,4-Triazoliumionen, 1,2,3-Triazoliumionen, Pyrrolidiniumionen, Chinoliniumionen, die gegebenenfalls in einer oder mehreren Seitenketten weitere funktionelle Gruppen wie Ether, Ester, Amide und / oder Carbamate tragen und die auch in oligomerer oder polymerer oder verbrückender Form vorliegen können, verstanden. Bevorzugte Organokationen K der Wertigkeit n auf Basis von Stickstoff sind beispielsweise Ammoniumionen, Pyridiniumionen, Pyridaziniumionen, Pyrimidiniumionen, Pyraziniumionen, Imidazoliumionen, Pyrrolidiniumionen, die gegebenenfalls in einer oder mehreren Seitenketten weitere funktionelle Gruppen wie Ether, Ester, Amide und / oder Carbamate tragen und die auch in oligomerer oder polymerer oder verbrückender Form vorliegen können. Besonders bevorzugte Organokationen K der Wertigkeit n auf Basis von Stickstoff sind beispielsweise Ammoniumionen, Pyridiniumionen und Imidazoliumionen, die gegebenenfalls in einer oder mehreren Seitenketten weitere funktionelle Gruppen wie Ether, Ester, Amide und / oder Carbamate tragen und die auch in oligomerer oder polymerer oder verbrückender Form vorliegen können. Auch polymere Kationen können unter den oben genannten Substitutionsmustern gemeint sein.

Unter einem beliebig substituierten Organokation K der Wertigkeit n auf Basis von Phosphor werden Phosphor(IV)-Verbindungen der Koordinationszahl 4 verstanden, wie beispielsweise beliebig substituierte Tetraalkyl-Phosphonium-, Trialkyl-Aryl-Phosphonium-, Dialkyl-Diaryl-Phosphonium-, Alkyl-Triaryl-Phosphonium-, Trialkylen-Aryl-Phosphonium-, Dialkylen-Diaryl-Phosphonium-, Alkylen-Triaryl-Phosphonium- oder Tetraaryl-Phosphonium-Salze, die gegebenenfalls in einer oder mehreren Seitenketten weitere funktionelle Gruppen wie Ether, Ester, Carbonyle, Amide und / oder Carbamate tragen und die auch in oligomerer oder polymerer oder verbrückender Form vorliegen können, verstanden. Ebenfalls umfasst sind 5 bis 8-gliedrige aliphatische, quasiaromatische oder aromatische cyclische Verbindungen, die 1, 2 oder 3 Phosphonium(IV)-Atome enthalten, die dann zur Herstellung der Vierbindigkeit des Phosphor-Atoms beliebig Alkyl- oder Aryl-substituiert sind. Aromatische Reste können zusätzlich beliebig viele Halogenatome, Nitro-, Cyano-, Trifluormethyl-, Ester-, und / oder Ether-Substituenten tragen. Selbstverständlich können die Alkyl- und Aryl-Reste untereinander durch Kohlenstoff- oder mono und / oder Polyether-Ketten verbrückt vorliegen, die dann mono- oder polycyclische Strukturen ausbilden. Bevorzugte Organokationen K der Wertigkeit n auf Basis von Phosphor sind beispielsweise beliebig substituierte Tetraalkyl-Phosphonium-, Trialkyl-Aryl-Phosphonium-, Dialkyl-Diaryl-Phosphonium-, Alkyl-Triaryl-Phosphonium- oder Tetraaryl-Phosphonium-Salze, die gegebenenfalls in einer oder mehreren Seitenketten weitere funktionelle Gruppen wie Carbonyle, Amide und / oder Carbamate tragen und die auch in oligomerer oder polymerer oder verbrückender Form vorliegen können, verstanden. Aromatische Reste können zusätzlich beliebig viele Halogenatome, Ester-, und / oder Ether-Substituenten tragen. Selbstverständlich können die Alkyl- und Aryl-Reste

untereinander durch Kohlenstoff- oder mono und / oder Polyether-Ketten verbrückt vorliegen, die dann mono- oder polycyclische Strukturen ausbilden. Besonders bevorzugte Organokationen K der Wertigkeit n auf Basis von Phosphor sind beispielsweise beliebig substituierte Tetraalkyl-Phosphonium-, Trialkyl-Aryl-Phosphonium-, Dialkyl-diaryl-Phosphonium-, Alkyl-Triaryl-Phosphonium- oder Tetraaryl-Phosphonium-Salze, die gegebenenfalls in einer oder mehreren Seitenketten weitere funktionelle Gruppen wie Carbonyle, Amide und / oder Carbamate tragen und die auch in oligomerer oder polymerer oder verbrückender Form vorliegen können, verstanden. Aromatische Reste können zusätzlich beliebig viele Halogenatome tragen. Selbstverständlich können die Alkyl- und Aryl-Reste untereinander durch Kohlenstoff- oder mono und / oder Polyether-Ketten verbrückt vorliegen, die dann mono- oder polycyclische Strukturen ausbilden. Auch polymere Kationen wie beispielsweise in US 3125555 genannt können unter den oben genannten Substitutionsmustern gemeint sein.

Unter einem beliebig substituierten Organokation K der Wertigkeit n auf Basis von Sauerstoff werden beispielsweise beliebig substituiertes Pyrylium, auch in annellierter Form wie im Benzopyrylium, Flavylium oder Naphthoxanthenium verstanden. Bevorzugte Organokationen K der Wertigkeit n auf Basis von Sauerstoff sind beispielsweise beliebig substituiertes Pyrylium, auch in annellierter Form wie im Benzopyrylium, Flavylium. Auch polymere Kationen können unter den oben genannten Substitutionsmustern gemeint sein.

Unter einem beliebig substituierten Organokation K der Wertigkeit n auf Basis von Schwefel werden Onium-Verbindungen des Schwefels verstanden, die gleiche oder verschiedene gegebenenfalls substituierte $C_1$- bis $C_{22}$-Alkyl-, $C_6$- bis $C_{14}$-Aryl-, $C_7$- bis $C_{15}$-Arylalkyl- oder $C_5$- bis $C_7$-Cycloalkylreste tragen und / oder oligomere oder polymere wiederkehrende verbindende Einheiten zu Sulfoniumsalzen mit $1 \leq n \leq 3$ aufbaut. Ebenfalls umfasst sind 5 bis 8-gliedrige aliphatische, quasiaromatische oder aromatische cyclische Verbindungen, die 1 oder 2 Sulfonium(III)-Atome enthalten, die dann zur Herstellung der Dreibindigkeit des Schwefel-Atoms beliebig Alkyl- oder Aryl-substituiert sind. Bevorzugte Organokationen K der Wertigkeit n auf Basis von Schwefel sind Onium-Verbindungen des Schwefels, die gleiche oder verschiedene gegebenenfalls substituierte $C_4$-bis $C_{14}$-Alkyl-, $C_6$- bis $C_{10}$-Aryl-, $C_7$- bis $C_{12}$-Arylalkyl- oder $C_5$- bis $C_6$-Cycloalkylreste tragen und / oder oligomere oder polymere wiederkehrende verbindende Einheiten zu Sulfoniumsalzen mit $1 \leq n \leq 3$ aufbauen sowie Thiopyrylium. Besonders bevorzugte Organokationen K der Wertigkeit n auf Basis von Schwefel sind Onium-Verbindungen des Schwefels, die gleiche oder verschiedene gegebenenfalls substituierte $C_6$- bis $C_{12}$-Alkyl-, $C_6$- bis $C_{10}$-Aryl-, $C_7$- bis $C_{12}$-Arylalkyl- oder $C_5$- bis $C_6$-Cycloalkylreste tragen und / oder oligomere oder polymere wiederkehrende verbindende Einheiten zu Sulfoniumsalzen mit $1 \leq n \leq 3$ aufbauen sowie Thiopyrylium. Auch polymere Kationen können unter den oben genannten Substitutionsmustern gemeint sein.

Unter einem beliebig substituierten Organokation K der Wertigkeit n auf Basis von Iod werden Onium-Verbindungen des Iods verstanden, die gleiche oder verschiedene gegebenenfalls substituierte $C_1$- bis $C_{22}$-Alkyl-, $C_6$- bis $C_{14}$-Aryl-, $C_7$- bis $C_{15}$-Arylalkyl- oder $C_5$- bis $C_7$-Cycloalkylreste tragen und / oder oligomere oder polymere wiederkehrende verbindende Einheiten zu Iodoniumsalzen mit $1 \leq n \leq 3$ aufbauen. Bevorzugte Organokationen K der Wertigkeit n auf Basis von Iod sind Onium-Verbindungen des Iods, die gleiche oder verschiedene gegebenenfalls substituierte $C_4$- bis $C_{14}$-Alkyl-, $C_6$- bis $C_{10}$-Aryl-, $C_7$- bis $C_{12}$-Arylalkyl- oder $C_5$- bis $C_6$-Cycloalkylreste tragen und / oder oligomere oder polymere wiederkehrende verbindende Einheiten zu Iodoniumsalzen mit $1 \leq n \leq 3$ aufbauen. Besonders bevorzugte Organokationen K der Wertigkeit n auf Basis von Iod sind Onium-Verbindungen des Iods, die gleiche oder verschiedene gegebenenfalls substituierte $C_4$-bis $C_{12}$-Alkyl-, $C_6$- bis $C_{10}$-Aryl-, $C_7$- bis $C_{12}$-Arylalkyl- oder $C_5$- bis $C_6$-Cycloalkylreste tragen und / oder oligomere oder polymere wiederkehrende verbindende Einheiten zu Iodoniumsalzen mit $1 \leq n \leq 3$ aufbauen. Auch polymere Kationen können unter den oben genannten Substitutionsmustern gemeint sein.

Weiterhin können Mischungen der oben genannten Kationen eingesetzt werden.

Unter einem aprotischen organischen Lösungsmittel oder ein Gemisch aprotischer organischer Lösungsmittel S werden nicht ohne starke Base deprotonierbare Lösungsmittel verstanden (Reichardt, C., Solvents and Solvent Effects in Organic Chemistry, 3. Aufl.; Wiley-VCH: Weinheim, (2003)). Beispiele für aprotische organische Lösungsmittel sind Alkane, Alkene, Alkine, Benzol und Aromaten mit aliphatischen und / oder aromatischen Substituenten, Carbonsäureester und / oder Ether. Bevorzugte aprotische organische Lösungsmittel sind Alkane, Aromaten mit aliphatischen und / oder aromatischen Substituenten und / oder Ether. Verwendet werden z.B. aromatische Kohlenwasserstoffe wie Solvent-Naphtha, Toluol oder Xylol oder Ether wie Tetrahydrofuran, Methyltetrahydrofuran, Diethylether oder Dimethoxyethan. Das Lösungsmittel sollte möglichst wasserfrei sein. In einer bevorzugten Ausführungsform unterscheidet sich das erste Lösungsmittel oder Lösungsmittelgemisch (Schritt i) von dem zweiten Lösungsmittel oder Lösungsmittelgemisch (Schritt iv), das in dem Verfahren eingesetzt wird. In einer bevorzugten Ausführungsform werden in Schritt (i) und Schritt (iv) die gleichen Lösungsmittel oder Lösungsmittelgemische eingesetzt.

Bevorzugt steht M für Magnesium, Calcium oder Aluminium und besonders bevorzugt steht M für Magnesium.

Bevorzugt steht X für ein Halogenid oder Alkoxid, besonders bevorzugt steht X für ein Halogenid und insbesondere bevorzugt steht X für Chlorid oder Bromid.

Bevorzugt steht Y für Brom oder Chlor und besonders bevorzugt steht Y für Brom.

Bevorzugt steht n für 1 oder 2 und besonders bevorzugt steht n für 1.

$C_1$-$C_{22}$-Alkyl steht im Rahmen der Erfindung beispielsweise für Methyl, Ethyl, n-Propyl, isoPropyl, n-Butyl,

sec.-Butyl, tert.-Butyl, n-Pentyl, 1-Methylbutyl, 2-Methylbutyl, 3-Methylbutyl, neo-Pentyl, 1-Ethylpropyl, 1,1-Dimethylpropyl, 1,2-Dimethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl oder 1-Ethyl-2-methylpropyl, n-Heptyl, n-Octyl, 2-Ethylhexyl, Pinakyl, n-Nonyl, n-Decyl, n-Dodecyl, n-Tridecyl, n-Tetradecyl, n-Hexadecyl, n-Octadecyl, n-Eicosyl, n-Docosyl. Gleiches gilt für den entsprechenden Alkylrest beispielsweise in Aralkyl- bzw. Alkylaryl-, Alkylphenyl- oder Alkylcarbonylresten.

Alkylenreste in substituierten Alkylresten sowie Alkenylreste oder Alkinylreste stehen beispielsweise für die den vorangehenden Alkylresten entsprechenden Alkylenreste, Alkenylreste oder Alkinylreste.

Beispiele sind 2-Chlorethyl, Benzyl, Allyl, 2-Buten-1-yl, Propargyl.

Cycloalkyl steht im Rahmen der Erfindung beispielsweise für Cyclopropyl, Cyclobutyl, Cyclopentyl, Cyclohexyl, Cycloheptyl, Adamantyl oder die isomeren Menthyle.

Aryl steht für einen carbocyclischen aromatischen Rest mit 6 bis 14 Gerüstkohlenstoffatomen. Gleiches gilt für den aromatischen Teil eines Arylalkylrestes, auch Aralkylrest genannt, sowie für Arylbestandteile komplexerer Gruppen, wie z.B. Arylcarbonylresten.

Beispiele für $C_6$- bis $C_{14}$-Aryl sind Phenyl, o-, p-, m-Tolyl, o-, p-, m-Ethylphenyl, Naphthyl, Phenanthrenyl, Anthracenyl, Fluorenyl, o-, p-, m-Fluorphenyl, o-, p-, m-Chlorphenyl, o-, p-, m-Methoxyphenyl, o-, p-, m-Trifluormethylphenyl, o-, p-, m-Trifluormethoxyphenyl, o-, m- oder p-Biphenylyl, o-, p-, m-Phenoxyphenyl, 3,4-Dimethylphenyl, 3,4-Dichlorphenyl, 3,4-Difluorphenyl, 3,4-Dimethoxyphenyl, 4-Methyl-3-fluorphenyl, 4-Methyl-3-chlorphenyl, 3,4,5-Trifluorphenyl.

Arylalkyl bzw. Aralkyl bedeutet jeweils unabhängig einen geradkettigen, cyclischen, verzweigten oder unverzweigten Alkyl-Rest nach vorstehender Definition, der einfach, mehrfach oder vollständig durch Aryl-Reste gemäß vorstehender Definition substituiert sein kann.

Beispiele sind Benzyl, 4-Chlorbenzyl, Phenethyl, 2-Phenyl-1-propyl, 3-Phenyl-1-propyl, 1-Phenyl-2-propyl, Diphenylmethyl.

Beispiele für eine 2-8 gliedrige gegebenenfalls durch Alkyl substituierte und / oder durch Sauerstoff-Atome unterbrochene Kohlenstoff-Brücke sind $-CH_2-CH_2-$, $-CH_2-CH_2-CH_2-$, $-CH_2-CH_2-CH_2-CH_2-$, $-CH_2-CH_2-O-CH_2-CH_2-$, $-CH_2-CH(CH_3)-$.

Beispiele für einen 4-14 gliedrigen gegebenenfalls durch Alkyl- oder Aryl- substituierten und / oder durch Sauerstoff-Atome unterbrochenen Kohlenstoff-Tricyclus sind:

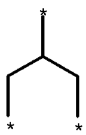 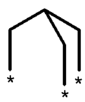 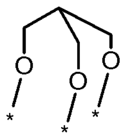 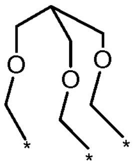 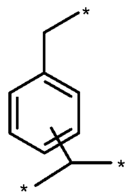 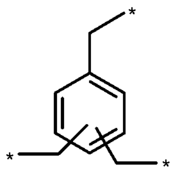

Die Umsetzung wird im allgemeinen ohne Anwendung von Überdruck ausgeführt. Praktisch wird man dann also bei mindestens dem Eigendruck arbeiten.

Die Umsetzung wird im allgemeinen bei Temperaturen von -20 °C bis 100 °C, bevorzugt von -10 °C bis 80 °C, besonders bevorzugt von 0 °C bis 70 °C und insbesondere bevorzugt von 10 °C bis 65 °C durchgeführt.

Die Umsetzung wird praktischerweise durch Zugabe von maximal 10 % der Gesamtmenge der Verbindung (III) gestartet und die Verbindung (III) wird anschließend als 10-90 %-ige, bevorzugt 15-75%-ige, besonders bevorzugt als 20-50 %-ige Lösung im Lösungsmittel S zugegeben, wobei sich die Temperatur T im oben beschriebenen Rahmen bewegt und die Reaktion durch gleichmäßige Dosierung der Verbindung (III) kontrolliert exotherm gehalten wird. Zum Anspringen der Reaktion können darüber hinaus optional die dem Fachmann bekannten Verbindungen wie Dibromethan oder Iod verwendet werden oder die Metalloberfläche M kann durch Ultraschall aktiviert werden.

Es ist von Vorteil, während der gesamten Dauer der Reaktion, z.B. durch Rühren, für eine gute Durchmischung des Ansatzes zu sorgen sowie durch Wahl eines geeigneten Lösungsmittels den Start der Reaktion gut zu ermöglichen.

Durch das erfindungsgemäße Verfahren werden deutlich bessere Ausbeuten im Vergleich zu den bekannten Verfahren erhalten, beispielsweise mindestens anderthalb mal so hohe Ausbeuten.

Ebenfalls offenbart sind die gemäß dem erfindungsgemäßen Verfahren herstellbaren oder hergestellten Triaryl-organoborate. Die so erhältlichen oder erhaltenen Triaryl-organoborate enthalten zudem bevorzugt < 10.000 ppm eines Tetraarylborates $R_4^3B^-$, besonders bevorzugt < 5.000 ppm eines Tetraarylborates $R_4^3B^-$ und insbesondere bevorzugt

< 1.000 ppm eines Tetraarylborates $R_4^3B^-$. Die Einheit ppm bezieht sich auf die Gewichtsanteile. Die Anwesenheit von Tetraarylboraten $R_4^3B^-$ führt bei längeren Lagerung zu einer eingeschränkten Beschreibbarkeit unbelichteter Photopolymerfilme enthaltend die erfindungsgemäßen Triaryl-organoborate bzw. einem Verlust der holographischen Eigenschaften in belichteten Photopolymerfilmen.

Weiterhin offenbart ist die Verwendung der erfindungsgemäß hergestellten Triaryl-organoborate in Photoinitiatorsystemen, sowie Photopolymerzusammensetzungen enthaltend eine photopolymerisierbare Komponente und ein Photoinitiatorsystem enthaltend die erfindungsgemäß hergestellten Triaryl-organoborate. Weiterhin werden aus den genannten Photopolymerzusammensetzungen holographische Medien und die daraus erhältlichen Hologramme zugänglich.

Geeignete Matrixpolymere einer entsprechenden Photopolymer-Formulierung können insbesondere vernetzt und besonders bevorzugt dreidimensional vernetzt sein.

Vorteilhaft ist auch, wenn die Matrixpolymere Polyurethane sind, wobei die die Polyurethane insbesondere durch Umsetzung wenigstens einer Polyisocyanat-Komponente a) mit wenigstens einer Isocyanat-reaktiven-Komponente b) erhältlich sein können.

Die Polyisocyanat-Komponente a) umfasst bevorzugt wenigstens eine organische Verbindung mit wenigstens zwei NCO-Gruppen. Bei diesen organischen Verbindungen kann es sich insbesondere um monomere Di- und Triisocyanate, Polyisocyanate und / oder NCO-funktionelle Prepolymere handeln. Die Polyisocyanat-Komponente a) kann auch Mischungen monomerer Di- und Triisocyanate, Polyisocyanate und / oder NCO-funktioneller Prepolymere enthalten oder daraus bestehen.

Als monomere Di- und Triisocyanate können alle dem Fachmann an sich gut bekannten Verbindungen oder deren Mischungen eingesetzt werden. Diese Verbindungen können aromatische, araliphatische, aliphatische oder cycloaliphatische Strukturen aufweisen. In untergeordneten Mengen können die monomeren Di- und Triisocyanate auch Monoisocyanate, d.h. organische Verbindungen mit einer NCO-Gruppe umfassen.

Geeignete Prepolymere enthalten Urethan- und / oder Harnstoff-Gruppen sowie gegebenenfalls weitere durch Modifizierung von NCO-Gruppen entstandene Strukturen wie oben genannt. Derartige Prepolymere sind beispielsweise durch Umsetzung der oben genannten monomeren Di- und Triisocyanate und / oder Polyisocyanaten a1) mit isocyanatreaktiven Verbindungen b1) erhältlich.

Als isocyanatreaktive Verbindungen b1) können Alkohole, Amino oder Mercapto-Verbindungen, bevorzugt Alkohole, verwendet werden. Dabei kann es sich insbesondere um Polyole handeln. Ganz besonders bevorzugt können als isocyanatreaktive Verbindung b1) Polyester-, Polyether-, Polycarbonat-, Poly(meth)acrylat- und / oder Polyurethan-Polyole verwendet werden.

Es ist ebenfalls möglich, als isocyanatreaktive Verbindungen b1) Amine einzusetzen. Beispiele geeigneter Amine sind Ethylendiamin, Propylendiamin, Diaminocyclohexan, 4,4'-Dicyclohexylmethandiamin, Isophorondiamin (IPDA), difunktionelle Polyamine wie z.B. die Jeffamine®, aminterminierte Polymere, insbesondere mit zahlenmittleren Molmassen ≤ 10.000 g/Mol. Mischungen der vorgenannten Amine können ebenfalls verwendet werden.

Es ist ebenfalls möglich, als isocyanatreaktive Verbindungen b1) Aminoalkohole einzusetzen. Beispiele geeigneter Aminoalkohole sind die isomeren Aminoethanole, die isomere Aminopropanole, die isomeren Aminobutanole und die isomeren Aminohexanole oder deren beliebige Mischungen.

Alle vorgenannten isocyanatreaktiven Verbindungen b1) können untereinander beliebig vermischt werden.

Bevorzugt ist auch, wenn die isocyanatreaktiven Verbindungen b1) eine zahlenmittlere Molmasse von ≥ 200 und ≤ 10.000 g/Mol, weiter bevorzugt ≥ 500 und ≤ 8.000 g/Mol und ganz besonders bevorzugt ≥ 800 und ≤ 5.000 g/Mol aufweisen. Die OH-Funktionalität der Polyole beträgt bevorzugt 1.5 bis 6.0, besonders bevorzugt 1.8 bis 4.0.

Die Prepolymere der Polyisocyanat-Komponente a) können insbesondere einen Restgehalt an freiem monomeren Di- und Triisocyanaten < 1 Gew.-%, besonders bevorzugt < 0.5 Gew.-% und ganz besonders bevorzugt < 0.3 Gew.-% aufweisen.

Es ist gegebenenfalls auch möglich, dass die Polyisocyanat-Komponente a) vollständig oder anteilsmäßig eine organische Verbindung enthält, deren NCO-Gruppen ganz oder teilweise mit aus der Beschichtungstechnologie bekannten Blockierungsmitteln umgesetzt sind. Beispiel für Blockierungsmittel sind Alkohole, Lactame, Oxime, Malonester, Pyrazole sowie Amine, wie z.B. Butanonoxim, Diisopropylamin, Malonsäurediethylester, Acetessigester, 3,5-Dimethylpyrazol, ε-Caprolactam, oder deren Mischungen.

Besonders bevorzugt ist, wenn die Polyisocyanat-Komponente a) Verbindungen mit aliphatisch gebundenen NCO-Gruppen umfasst, wobei unter aliphatisch gebundenen NCO-Gruppen derartige Gruppen verstanden werden, die an ein primäres C-Atom gebunden sind. Die isocyanatreaktive Komponente b) umfasst bevorzugt wenigstens eine organische Verbindung, die im Mittel wenigstens 1.5 und bevorzugt 2 bis 3 isocyanatreaktive Gruppen aufweist. Im Rahmen der vorliegenden Erfindung werden als isocyanatreaktive Gruppen bevorzugt Hydroxy-, Amino- oder MercaptoGruppen angesehen.

Die isocyanatreaktive Komponente kann insbesondere Verbindungen umfassen, die im Zahlenmittel wenigstens 1.5 und bevorzugt 2 bis 3 isocyanatreaktive Gruppen aufweisen.

Geeignete polyfunktionelle, isocyanatreaktive Verbindungen der Komponente b) sind beispielsweise die oben beschriebenen Verbindungen b1).

Bei einer weiteren bevorzugten Ausführungsform ist vorgesehen, dass das Schreibmonomer c) wenigstens ein mono- und / oder ein multifunktionales Schreibmonomer umfasst oder daraus besteht. Weiter bevorzugt kann das Schreibmonomer wenigstens ein mono- und / oder ein multifunktionelles (Meth)acrylat-Schreibmonomere umfassen oder daraus bestehen. Ganz besonders bevorzugt kann das Schreibmonomer wenigstens ein mono- und / oder ein multifunktionelles Urethan-(meth)acrylat umfassen oder daraus bestehen.

Photoinitiatoren der Komponente d) sind üblicherweise durch aktinische Strahlung aktivierbare Verbindungen, die eine Polymerisation der Schreibmonomere auslösen können. Bei den Photoinitiatoren kann zwischen unimolekularen (Typ I) und bimolekularen (Typ II) Initiatoren unterschieden werden. Des Weiteren werden sie je nach ihrer chemischen Natur in Photoinitiatoren für radikalische, anionische, kationische oder gemischte Art der Polymerisation unterschieden.

Typ I-Photoinitiatoren (Norrish-Typ-I) für die radikalische Photopolymerisation bilden beim Bestrahlen durch eine unimolekulare Bindungsspaltung freie Radikale. Beispiele für Typ I-Photoinitiatoren sind Triazine, Oxime, Benzoinether, Benzilketale, Bis-imidazole, Aroylphosphinoxide, Sulfonium- und Iodoniumsalze.

Typ II-Photoinitiatoren (Norrish-Typ-II) für die radikalische Polymerisation bestehen aus einem Farbstoff als Sensibilisator und einem Coinitiator und durchlaufen bei der Bestrahlung mit auf den Farbstoff angepasstem Licht eine bimolekulare Reaktion. Zunächst absorbiert der Farbstoff ein Photon und überträgt aus einem angeregten Zustand Energie auf den Coinitiator. Dieser setzt durch Elektronen- oder Protonentransfer oder direkte Wasserstoffabstraktion die polymerisationsauslösenden Radikale frei.

Im Sinne dieser Erfindung werden bevorzugt Typ II-Photoinitiatoren verwendet.

Solche Photoinitiatorsysteme sind prinzipiell in der EP 0 223 587 A beschrieben und bestehen bevorzugt aus einer Mischung von einem oder mehreren Farbstoffen mit den erfindungsgemäßen Verbindungen der Formel (IV).

Geeignete Farbstoffe, die zusammen mit einer Verbindung der Formel (IV) einen Typ II-Photoinitiator bilden, sind die in der WO 2012062655 beschriebenen kationischen Farbstoffe in Kombination mit den eben dort beschriebenen Anionen.

Die offenbarten Triarylalkylborate werden vorteilhafterweise als Coinitiatoren eingesetzt.

Das Photoinitiatorsystem kann außerdem einen weiteren Co-Initiator enthalten wie beispielsweise Trichloromethylinitiatoren, Aryloxidinitiatoren, Bisimidazolinitiatoren, Ferroceninitiatoren, Aminoalkylinitiatoren, Oximinitiatoren, Thiolinitiatoren oder Peroxideinitiatoren.

Ebenfalls ist es möglich, dass die Photopolymer-Formulierung zusätzlich Urethane als Additive enthält, wobei die Urethane insbesondere mit wenigstens einem Fluoratom substituiert sein können.

Ebenfalls offenbart ist ein Photopolymer enthaltend eine Photopolymer-Formulierung, insbesondere umfassend Matrixpolymere, ein Schreibmonomer und einen Photoinitiatorsystem, das eine Verbindung erhältlich oder erhalten nach dem erfindungsgemäßen Verfahren umfasst.

Ebenfalls zugänglich wird ein holographisches Medium insbesondere in Form eines Films, enthaltend ein offenbartes Photopolymer oder erhältlich unter Verwendung einer offenbarten Photopolymer-Formulierung. Außerdem ist die Photopolymerzusammensetzung verwendbar zur Herstellung holographischer Medien.

In solch ein holographisches Medium können holographische Informationen einbelichtet werden.

Die offenbarten holographischen Medien können durch entsprechende Belichtungsprozesse für optische Anwendungen im gesamten sichtbaren und nahen UV-Bereich (300-800 nm) zu Hologrammen verarbeitet werden. Visuelle Hologramme umfassen alle Hologramme, die nach dem Fachmann bekannten Verfahren aufgezeichnet werden können. Darunter fallen unter anderem In-Line (Gabor) Hologramme, Off-Axis Hologramme, Full-Aperture Transfer Hologramme, Weißlicht-Transmissionshologramme ("Regenbogenhologramme), Denisyukhologramme, Off-Axis Reflektionshologramme, Edge-Lit Hologramme sowie holographische Stereogramme. Bevorzugt sind Reflektionshologramme, Denisyukhologramme, Transmissionshologramme.

Mögliche optische Funktionen der Hologramme, die mit den offenbarten Photopolymer-Formulierungen hergestellt werden können entsprechen den optische Funktionen von Lichtelementen wie Linsen, Spiegel, Umlenkspiegel, Filter, Streuscheiben, Beugungselemente, Diffusoren, Lichtleiter, Lichtlenker (waveguides), Projektionsscheiben und / oder Masken. Ebenfalls können Kombinationen aus diesen optischen Funktionen unabhängig voneinander in einem Hologramm vereinigt sein. Häufig zeigen diese optischen Elemente eine Frequenzselektivität, je nachdem wie die Hologramme belichtet wurden und welche Dimensionen das Hologramm hat.

Zudem können mittels der holographischen Medien auch holographische Bilder oder Darstellungen hergestellt werden, wie zum Beispiel für persönliche Portraits, biometrische Darstellungen in Sicherheitsdokumenten, oder allgemein von Bilder oder Bildstrukturen für Werbung, Sicherheitslabels, Markenschutz, Markenbranding, Etiketten, Designelementen, Dekorationen, Illustrationen, Sammelkarten, Bilder und dergleichen sowie Bilder, die digitale Daten repräsentieren können u.a. auch in Kombination mit den zuvor dargestellten Produkten. Holographische Bilder können den

Eindruck eines dreidimensionalen Bildes haben, sie können aber auch Bildsequenzen, kurze Filme oder eine Anzahl von verschiedenen Objekten darstellen, je nachdem aus welchem Winkel, mit welcher (auch bewegten) Lichtquelle etc. diese beleuchtet wird. Aufgrund dieser vielfältigen Design-Möglichkeiten stellen Hologramme, insbesondere Volumenhologramme, eine attraktive technische Lösung für die oben genannten Anwendung dar.

Die holographischen Medien können zur Aufzeichnung von In-Line, Off-Axis, Full-Aperture Transfer, Weißlicht-Transmissions, Denisyuk, Off-Axis Reflektions oder Edge-Lit Hologrammen sowie holographischen Stereogrammen, insbesondere zur Herstellung von optischen Elementen, Bildern oder Bilddarstellungen verwendet werden.

Hologramme werden aus den offenbarten holographischen Medien zugänglich.

Die folgenden Beispiele dienen der beispielhaften Erläuterung der Erfindung, ohne sie auf diese zu beschränken.

## Beispiele

Messmethoden:

| | |
|---|---|
| OH Zahl: | Die angegebenen OH-Zahlen wurden gemäß DIN 53240-2 bestimmt. |
| NCO-Wert: | Die angegebenen NCO-Werte (Isocyanat-Gehalte) wurden gemäß DIN EN ISO 11909 bestimmt. |
| Festkörpersehalt: | Die angegebenen Festkörpergehalte wurden gemäß DIN EN ISO 3251 bestimmt. |
| Brechunssindexmodulation Δn: | Die holographische Eigenschaften Δn der holographischen Medien wurden mittels Zweistrahlinterferenz in Reflexionsanordnung wie in WO2015091427 beschrieben bestimmt, ein repräsentatives Ergebnis ist in Figur 1 abgebildet. |

## Substanzen:

Die verwendeten Lösungsmittel, Reagenzien sowie alle Bromaromaten wurden im Chemikalienhandel bezogen. Wasserfreie Lösungsmittel enthalten < 50 ppm Wasser.

| | |
|---|---|
| Ethylboronsäure-Pinacolester | [82954-89-0] ist bei der TCI Europe N.V., Zwijndrecht, Belgien erhältlich. |
| Isopropylboronsäure-Pinacolester | [76347-13-2] ist bei der ABCR GmbH & Co. KG, Karlsruhe, Deutschland erhältlich. |
| 2-Isopropenylboronsäure-Pinacolester | [126726-62-3] ist bei der ABCR GmbH & Co. KG, Karlsruhe, Deutschland erhältlich. |
| 1-Dodecylboronsäure-Pinacolester | [177035-82-4] ist bei der ABCR GmbH & Co. KG, Karlsruhe, Deutschland erhältlich. |
| 3-Phenyl-1-propylboronsäure-Pinacolester | [329685-40-7] ist bei der ABCR GmbH & Co. KG, Karlsruhe, Deutschland erhältlich. |
| Diisopropylallylboronat | [51851-79-7] ist bei der ABCR GmbH & Co. KG, Karlsruhe, Deutschland erhältlich. |
| (1,3,2-Dioxaborinan-2-yl) cyclohexan | [30169-75-6] ist bei der ABCR GmbH & Co. KG, Karlsruhe, Deutschland erhältlich. |
| Dibromboran-Dimethyl-sulfid-Komplex | [55671-55-1] ist bei der Aldrich Chemie, Steinheim, Deutschland erhältlich. |
| 1-Octadecen | [112-41-4] ist bei der ABCR GmbH & Co. KG, Karlsruhe, Deutschland erhältlich. |
| Desmorapid Z | Dibutylzinn-dilaurat [77-58-7], Produkt der Covestro AG, Leverkusen, Deutschland. |
| Desmodur® N 3900 | Produkt der Covestro AG, Leverkusen, DE, He-xandiisocyanat-basiertes Polyisocyanat, Anteil an Imino¬oxa-diazindion mindestens 30 %, NCO-Gehalt: 23.5 %. |
| Fomrez UL 28 | Urethanisierungskatalysator, Handelsprodukt der Momentive Performance Chemicals, Wilton, CT, USA. |

2-Hexyl-1,3,2-dioxaborinan [86290-24-6] wurde wie in Organometallics 1983, 2 (10), S. 1311-16, DOI:10.1021/om50004a008 beschrieben aus 1-Hexen, 1,3-Propandiol und Dibromboran-Dimethylsulfid-Komplex hergestellt.

2-Octadecyl-4,4,5,5-tetramethyl-1,3,2-dioxaborolan wurde analog zu Organometallics 1983, 2 (10), S. 1311-16, DOI:10.1021/om50004a008 aus 1-Octadecen, 1,3-Propandiol und Dibromboran-Dimethylsulfid-Komplex hergestellt.

Farbstoff 1 (3,7-Bis(diethylamino)-phenoxazin-5-ium bis(2-ethylhexyl)sulfobernsteinsäureester) wurde wie in WO 2012062655 beschrieben hergestellt.

Polyol 1 wurde wie Polyol 1 in der WO2015091427 beschrieben hergestellt.

Urethanacrylat 1, (Phosphorothioyltris(oxybenzol-4,1-diylcarbamoyloxyethan-2,1-diyl)trisacrylat, [1072454-85-3]) wurde wie in WO2015091427 beschrieben hergestellt.

Urethanacrylat 2, (2-({[3-(Methylsulfanyl)phenyl]carbamoyl}oxy)-ethylprop-2-enoat, [1207339-61-4]) wurde wie in WO2015091427 beschrieben hergestellt.

Additiv 1, Bis(2,2,3,3,4,4,5,5,6,6,7,7-dodecafluorheptyl)-(2,2,4-trimethylhexan-1,6-diyl)biscarbamat [1799437-41-4] wurde wie in WO2015091427 beschrieben hergestellt.

**Herkunft der verwendeten Kationen 1/n $K^{n+}$:**

Die in der Tabelle 1 genannten Kationen 1/n $K^{n+}$ wurden von dem genannten Chemikalienhändler bezogen oder gemäß der genannten Quelle hergestellt oder die Herstellung ist nachfolgend beschrieben.

Tabelle 1: Übersicht der verwendeten Kationen der Formel (II)

| **Index** | **Name** | **Struktur** | CAS | **Quelle** |
|---|---|---|---|---|
| K-1 | Tetramethylammonium bromid | $Me_4NBr$ | [64-20-0] | 426296, Aldrich |
| K-2 | Tetrabutylammonium bromid | $Bu_4NBr$ | [1643-19-2] | 426288, Aldrich |
| K-3 | Benzyldimethylhexadecylammonium chlorid | $BnMe_2HexadecylNCl$ | [122-18-9] | AB252026, abcr GmbH |
| K-4 | Hexadecyltrimethylammonium bromid | 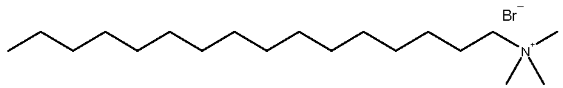 | [57-09-0] | AB 117004, abcr GmbH |
| K-5 | Benzethonium chlorid | 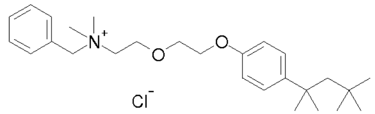 | [121-54-0] | AB131627, abcr GmbH |
| K-6 | 1-Hexadecyl-3-methylimidazolium chlorid | 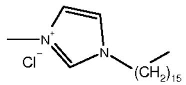 | [61546-01-8] | AB289677, abcr GmbH |
| K-7 | 1-Methyl-3-octylimidazolium chlorid | 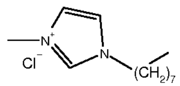 | [64697-40-1] | AB289637, abcr GmbH |
| K-8 | 1-Dodecyl-3-methylimidazolium chlorid | 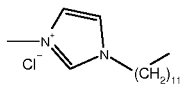 | [114569-84-5] | AB289681, abcr GmbH |
| K-9 | 1-Ethyl-3-imidazolium chlorid | 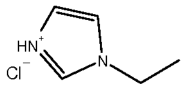 | [81505-35-3] | AB289800, abcr GmbH |
| K-10 | 1-Benzyl-3-hexadecyl-imidazolium bromid | 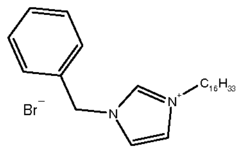 | [1224595-52-1] | Fuel Processing Technology (2014), 118,296-301. |

(fortgesetzt)

| Index | Name | Struktur | CAS | Quelle |
|---|---|---|---|---|
| K-11 | N-Hexadecylpyridinium chloride monohydrat | 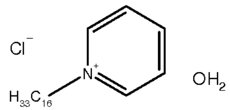 | [6004-24-6] | AB117002, abcr GmbH |
| K-12 | N-Docosylpyridinium bromid | 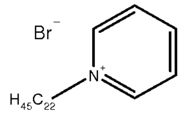 | [80039-83-4] | J. Am. Chem. Soc. (2002), 124 (11), 2604-2613. |
| K-13 | 4-tert.-Butyl-1-hexadecyl-pyridinium chlorid | 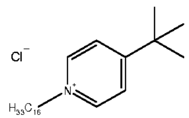 | [1702465-32-4] | WO 2015055576 A1 |
| K-14 | 1,1'-[1,3-Phenylenebis(methylen)] bis-pyridinium dichlorid | 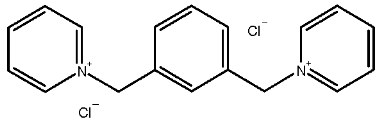 | [84002-71-1] | Zhurnal Neorganicheskoi Khimii (1978), 23, (3), 825-6. |
| K-15 | N,N,N,N',N',N'-Hexaethyl-1,3-benzendimethan aminium dibromid | 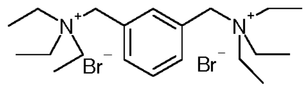 | [66753-59-1P] | Chemische Berichte (1984), 117 (4), 1487-96 |
| K-16 | N,N-Dioctadecyl-piperidinium chlorid | 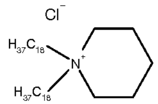 | [61550-95-6] | J. Am. Chem. Soc. (1955), 77, 485-6. |
| K-17 | N-Hexadecyl-N,N-dimethyl-anilinium bromid | 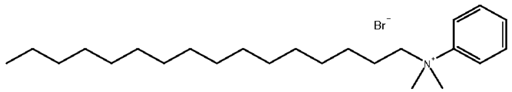 | [17695-00-0] | Taiwan Kexue (1959), 13, 95-8. |
| K-18 | N,N,N,N',N',N'-Hexabutyl-hexamethylendiammonium dibromid | 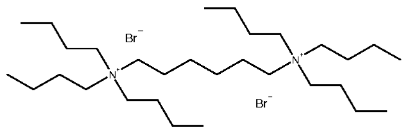 | [745829-82-7P] | Chemical Engineering Science, 69 (1), 483-491 |

(fortgesetzt)

| Index | Name | Struktur | CAS | Quelle |
|---|---|---|---|---|
| K-19 | N-(2-(Benzoyloxy)ethyl)-N,N-dimethyloctadecan-1-aminium bromid | 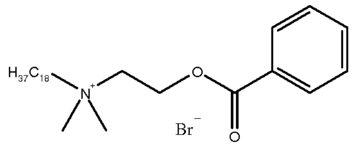 | [152167-30-1] | US 5194472 A |
| K-20 | N-(2-((2-Ethylhexanoyl)oxy)ethyl)-N,N-dimethylhexadecan-1-aminium bromid | 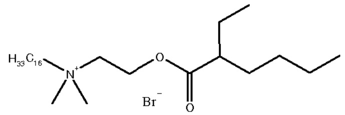 | | Herstellvorschrift siehe unten |
| K-21 | N-Benzyl-N,N-dimethyl-2-(2-(palmitoyloxy)ethoxy) ethan-1-aminium bromid | 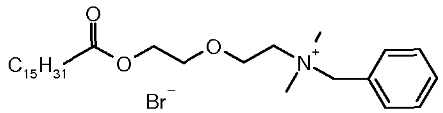 | | Herstellvorschrift siehe unten |
| K-22 | 2-(Benzoyloxy)-N,N-dimethyl-N-(2-(palmitoyloxy) ethyl)ethan-1-aminium bromid | 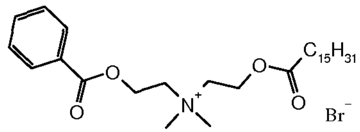 | | Herstellvorschrift siehe unten |
| K-23 | N-(3-((2-Ethylhexyl)oxy)-3-oxopropyl)-N,N-dimethyloctadecan-1-aminium bromid | 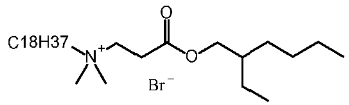 | | Herstellvorschrift siehe unten |
| K-24 | N-(2-((Hexylcarbamoyl)oxy)ethyl)-N,N-dimethylhexadecan-1-aminium bromid | 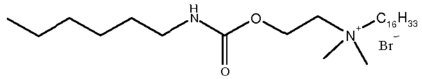 | | Herstellvorschrift siehe unten |
| K-25 | $N^1$,$N^{16}$-Dihexadecyl-$N^1$,$N^1$,$N^{16}$,$N^{16}$,7,7,10-heptamethyl-4,13-dioxo-3,14-dioxa-5,12-diazahexadecan-1,16-diaminium dibromid | 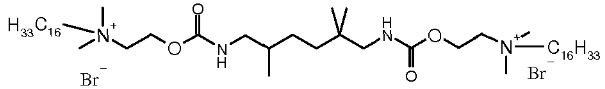 | | Herstellvorschrift siehe unten |
| K-26 | N-(2-((((5-(((2-(Hexadecyldimethylammonio) ethoxy)-carbonyl)amino)-1,3,3-trimethylcyclohexyl) methyl)-carbamoyl)oxy)ethyl)-N,N-dimethylhexadecan-1-aminium dibromid | 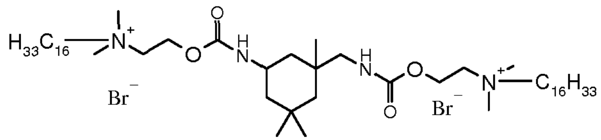 | | Herstellvorschrift siehe unten |

(fortgesetzt)

| Index | Name | Struktur | CAS | Quelle |
|---|---|---|---|---|
| K-27 | N,N'-(((((Methylenebis(cyclohexan-4,1-diyl))bis(azanediyl))bis(carbonyl))bis(oxy))bis(ethane-2,1-diyl))-bis(N,N-dimethylhexadecan-1-aminium) dibromid | 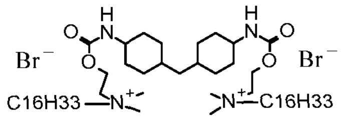 | | Herstellvorschrift siehe unten |
| K-28 | N,N'-(((((4-Methyl-1,3-phenylen)bis(azanediyl))-bis(carbonyl))bis(oxy))bis(ethan-2,1-diyl))bis(N,N-dimethylhexadecan-1-aminium) dibromid | 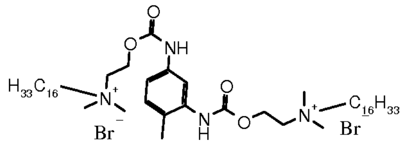 | [1073535-73-5] | Herstellvorschrift siehe unten |
| K-29 | N,N'-(((((Methylenbis(4,1-phenylen))bis(azanediyl))-bis(carbonyl))bis(oxy))bis(ethan-2,1-diyl))bis(N,N-dimethylhexadecan-1-aminium) dibromid | 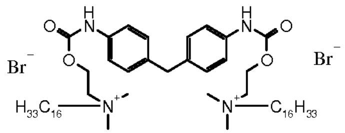 | | Herstellvorschrift siehe unten |
| K-30 | N-(2-(2-((Hexylcarbamoyl)oxy)ethoxy)ethyl)-N,N-dimethylhexadecan-1-aminium bromid | 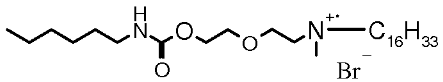 | | Herstellvorschrift siehe unten |
| K-31 | $N^1,N^{22}$-Dihexadecyl-$N^1,N^1,N^{22},N^{22}$,10,10,13-heptamethyl-7,16-dioxo-3,6,17,20-tetraoxa-8,15-diazadocosan-1,22-diaminium dibromid | 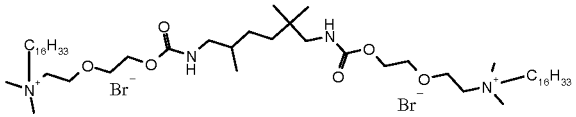 | | Herstellvorschrift siehe unten |
| K-32 | N-(2-(2-(((3-(10,10-Dimethyl-3-oxo-4,7-dioxa-2,10-diazahexacosan-10-ium-1-yl)-3,5,5-trimethyl-cyclohexyl)carbamoyl)oxy)ethoxy)ethyl)-N,N-dimethylhexadecan-1-aminium dibromid | 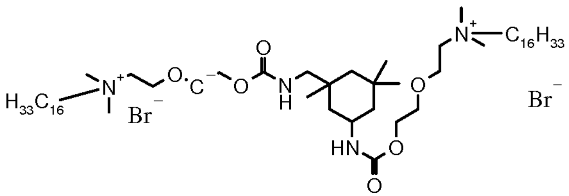 | | Herstellvorschrift siehe unten |
| K-33 | N,N'-((((((Methylenbis(cyclohexan-4,1-diyl))bis(azanediyl))bis(carbonyl))bis(oxy))bis(ethan-2,1-diyl))bis(oxy))bis(ethan-2,1-diyl))bis(N,N-dimethylhexadecan-1-aminium) dibromid | 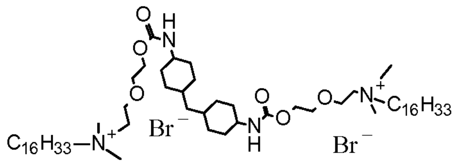 | | Herstellvorschrift siehe unten |

(fortgesetzt)

| Index | Name | Struktur | CAS | Quelle |
|---|---|---|---|---|
| K-34 | N,N'-(((((((4-Methyl-1,3-phenylen)bis(azanediyl)) bis-(carbonyl))bis(oxy))bis(ethan-2,1-diyl))bis(oxy)) bis(ethan-2,1-diyl))bis(N,N-dimethylhexadecan-1-aminium) dibromid | 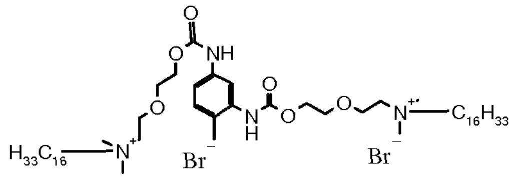 | | Herstellvorschrift siehe unten |
| K-35 | N,N'-(((((((Methylenbis(4,1-phenylen))bis (azandiyl))-bis(carbonyl))bis(oxy))bis(ethan-2,1-diyl)) bis-(oxy))bis(ethan-2,1-diyl))bis(N,N-dimethylhexadecan-1-aminium) dibromid | 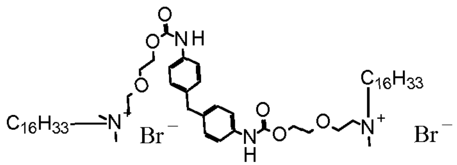 | | Herstellvorschrift siehe unten |
| K-36 | *N*[1],*N*[1],*N*[1],*N*[3],*N*[3],*N*[3],*N*[5],*N*[5],*N*[5]-Nonamethyl-1,3,5-benzentrimethanaminium triiodid | 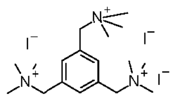 | 88888-13-5 | Chem. Ber. 117, 1487-1496 (1984). |
| K-37 | *N*-[2-[2-[2-(Benzoyloxy)ethoxy]etboxy]ethyl]-*N*,*N*-dimethyl-octadecan-1-amminium bromid | 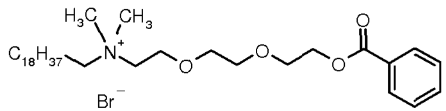 | [215591-20-1] | SK 278487 |
| K-38 | 1-[3-[(2-Ethylhexyl)oxy]-3-oxopropyl]-4-methyl-pyridinium chlorid | 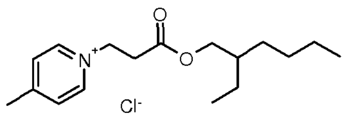 | [110250-87-8] | US 2857310 |
| K-39 | 4-Methyl-1-[2-[(1-oxotetradecyl)oxy]ethyl]-pyridinium chlorid | 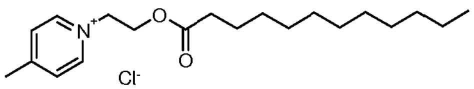 | [42936-94-7] | Izvestiya Vysshikh Uchebnykh Zavedenii, Khimiya i Khimicheskaya Tekhnologiya (1973), 16(6), 891. |

(fortgesetzt)

| Index | Name | Struktur | CAS | Quelle |
|---|---|---|---|---|
| K-40 | 1-[[3-(4-Methoxyphenyl)-2-oxo-5-oxazolidinyl] methyl]-4-phenyl-pyridinium bromid | 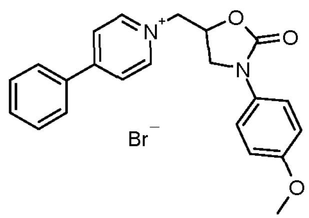 | [121082-85-7] | DE 3723797 |
| K-41 | 4-Methyl-1-[2-[(1-oxododecyl)amino] ethyl]-pyridinium chlorid | 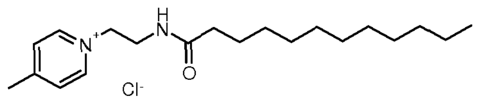 | [15147-43-0] | Chemicky Prumysl (1967), 17(2), 67-9. |
| K-42 | 4,5-Dihydro-1-methyl-3-[2-[(1-oxotetradecyl) oxy]-ethyl]-2-pentadecyl-1H-imidazolium chlorid | 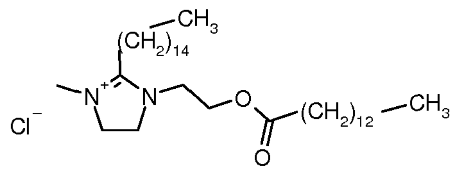 | [131625-89-3] | US 4954635 |
| K-43 | Morpholinium, 4,4-Bis[2-[[(9Z)-1-oxo-9-octadecen-1-yl]oxy]ethyl]-morpholinium chlorid | 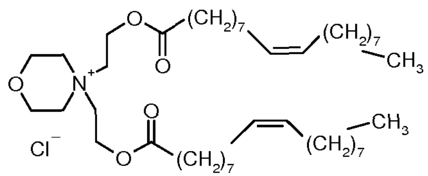 | [272462-96-1] | WO2000030444 |
| K-100 | Tributyltetradecylphosphonium chlorid | 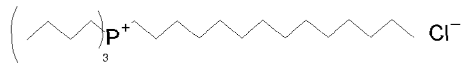 | [81741-28-8] | P444(14) Cl, Ionic Liquids Technologies GmbH |
| K-101 | Trihexyltetradecylphosphonium chlorid | 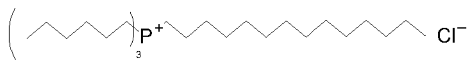 | [258864-54-9] | P666(14) Cl, Ionic Liquids Technologies GmbH |
| K-102 | Methyltriphenylphosphonium chlorid | 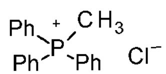 | [1031-15-8] | AB349191, abcr GmbH |

(fortgesetzt)

| Index | Name | Struktur | CAS | Quelle |
|---|---|---|---|---|
| K-103 | Triphenylbenzylphosphonium chlorid | 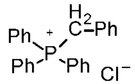 | [1100-88-5] | AB113982, abcr GmbH |
| K-104 | Tetraphenylphosphonium chlorid | 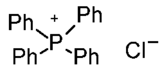 | [2001-45-8] | AB119018, abcr GmbH |
| K-105 | Allyltriphenylphosphonium bromid | 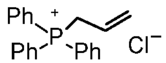 | [1560-54-9] | AB121395, abcr GmbH |
| K-106 | (2-Oxo-2-phenyl-ethyl)-triphenyl-phosphonium bromid | 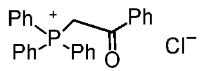 | [6048-29-9] | AB233312, abcr GmbH |
| K-200 | 4-Methyl-2,6-diphenylpyrylium tetrafluoroborat | 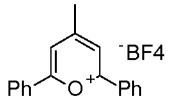 | [2340-23-0] | S919802, Aldrich |
| K-201 | 2,4,6-Triphenylpyrylium tetrafluoroborat | 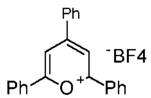 | [448-61-3] | AB119969, abcr GmbH |
| K-202 | 2,4,6-Trimethylpyrylium tetrafluoroborat | 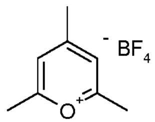 | [773-01-3] | AB177250, abcr GmbH |
| K-203 | 4,4'-Bis(2,6-diphenylpyrylium tetrafluoroborat) | 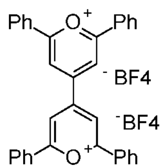 | [42559-29-5] | S873950, Aldrich |
| K-300 | Diphenyl[4-(phenylthio)phenyl]sulfonium hexafluorophosphat | 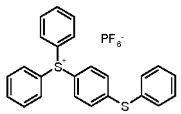 | [75482-18-7] | AB334122, abcr GmbH |

(fortgesetzt)

| Index | Name | Struktur | CAS | Quelle |
|---|---|---|---|---|
| K-301 | Bis(diphenylsulfonium)diphenylthioether hexafluorophosphat | 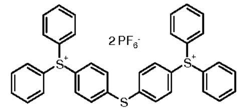 | [74227-35-3] | AB134315, abcr GmbH |
| K-302 | Diphenyl[4-(phenylthio)phenyl]sulfonium triflat | 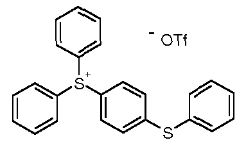 | [111281-12-0] | AB231614, abcr GmbH |
| K-303 | Mischung K-300 + K-301 (Cyracure UVI 6990) | | [104558-95-4] | BASF SE, Ludwigshafen, Germany |
| K-304 | Tris[4-[(4-acetylphenyl)thio]phenyl]sulfonium tris[(trifluoromethyl)sulfonyl] methanid | 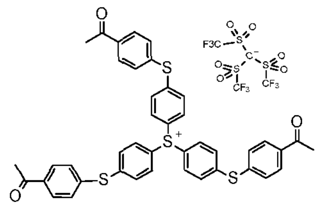 | [953084-20-3] | BASF SE, Ludwigshafen, Germany |
| K-305 | 2,4,6-Triphenyl thiopyrylium perchlorat | 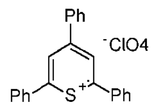 | [2930-37-2] | AKOS001017031, AKos GmbH Austr. 26 Steinen, D-79585 Germany |
| K-400 | (4-Methylphenyl)[4-(2-methylpropyl)phenyl iodonium] hexafluorophosphat | 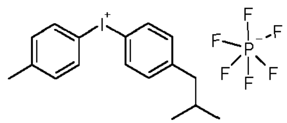 | [344562-80-7] | BASF SE, Ludwigshafen, Germany |
| K-401 | Diphenyliodonium chlorid | 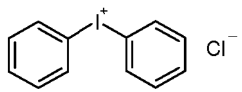 | [1483-72-3] | AB109613, abcr GmbH |

(fortgesetzt)

| Index | Name | Struktur | CAS | Quelle |
|---|---|---|---|---|
| K-402 | Bis(4-methylphenyl)iodonium hexafluorophosphat | 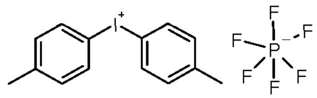 | [60565-88-0] | AB336755, abcr GmbH |
| K-403 | Diphenyleneiodonium chlorid | 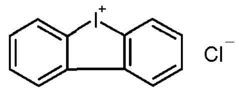 | [4673-26-1] | AB348986, abcr GmbH |

**Herstellung nicht kommerziell erhältlicher Kationen 1/n $K^{n+}$:**

**N-(2-((2-Ethylhexanoyl)oxy)ethyl)-N,N-dimethylhexadecan-1-aminium bromid (K-20)**

5.00 g N,N-Dimethylethanolamin wurden in trockenem Chloroform mit Eisbadkühlung vorgelegt und 9.12 g 2-Ethylhexansäurechlorid wurden vorsichtig zugetropft und 30 min bei Raumtemperatur nachgerührt. 30 mL gesättigte Natriumhydrogencarbonatlösung Lösung wurden zugegeben und die organische Lösung wurde so lange mit gesättigter Natriumhydrogencarbonatlösung extrahiert bis diese Chlorid-frei ist. Anschließend wurde die organische Phase mit 30 mL Wasser gewaschen, die Lösung getrocknet und das Lösungsmittel im Vakuum abdestilliert. Es wurden 10.99 g Aminoester erhalten.

Zu einer Lösung aus 10.99 g Aminoester in 30 mL Acetonitril wurden 15.58 g 1-Bromhexadecan gegeben und für 6 h am Rückfluss erhitzt. Das Lösungsmittel wurde fast vollständig im Vakuum abdestilliert, der ausgefallene Feststoff wurde isoliert und man erhielt 19.33 g eines farblosen klebrigen Harzes.

**N-Benzyl-N,N-dimethyl-2-(2-(palmitoyloxy)ethoxy)ethan-1-aminium bromid (K-21)**

6.00 g N,N-2-[2-(Dimethylamino)ethoxy]ethanol wurden in trockenem Chloroform mit Eisbadkühlung vorgelegt und 12.38 g Hexadecansäurechlorid wurden vorsichtig zugetropft und 30 min bei Raumtemperatur nachgerührt. 30 mL gesättigte Natriumhydrogencarbonatlösung Lösung wurden zugegeben und die organische Lösung wurde so lange mit gesättigter Natriumhydrogencarbonatlösung extrahiert bis diese Chlorid-frei ist. Anschließend wurde die organische Phase mit 30 mL Wasser gewaschen, die Lösung getrocknet und das Lösungsmittel im Vakuum abdestilliert. Es wurden 14.68 g Aminoester erhalten.

Zu einer Lösung aus 14.68 g Aminoester in 30 mL Acetonitril wurden 6.77 g Benzylbromid gegeben und für 6 h am Rückfluss erhitzt. Das Lösungsmittel wurde fast vollständig im Vakuum abdestilliert, der ausgefallene Feststoff wurde isoliert und man erhielt 8.23 g eines farblosen klebrigen Harzes.

**2-(Benzoyloxy)-N,N-dimethyl-N-(2-(palmitoyloxy)ethyl)ethan-1-aminium bromid (K-22)**

6.00 g N,N-2-[2-(Dimethylamino)ethoxy]ethanol wurden in trockenem Chloroform mit Eisbadkühlung vorgelegt und 12.38 g Hexadecansäurechlorid wurden vorsichtig zugetropft und 30 min bei Raumtemperatur nachgerührt. 30 mL gesättigte Natriumhydrogencarbonatlösung Lösung wurden zugegeben und die organische Lösung wurde so lange mit gesättigter Natriumhydrogencarbonatlösung extrahiert bis diese Chlorid-frei ist. Anschließend wurde die organische Phase mit 30 mL Wasser gewaschen, die Lösung getrocknet und das Lösungsmittel im Vakuum abdestilliert. Es wurden 14.68 g Aminoester erhalten.

Zu einer Lösung aus 14.68 g Aminoester in 30 mL Acetonitril wurden 6.77 g Benzylbromid gegeben und für 6 h am Rückfluss erhitzt. Das Lösungsmittel wurde fast vollständig im Vakuum abdestilliert, der ausgefallene Feststoff wurde isoliert und man erhielt 8.23 g eines farblosen klebrigen Harzes.

**N-(3-((2-Ethylhexyl)oxy)-3-oxopropyl)-N,N-dimethyloctadecan-1-aminium bromid (K-23)**

Zu einer Lösung aus 10.0 g N,N-Dimethyl-β-Alanine-2-ethylhexylester ([184244-48-2], hergestellt wie in US5565290 A beschrieben) in 30 mL Acetonitril wurden 15.0 g Octadecylbromid gegeben und für 6 h am Rückfluss erhitzt. Das Lösungsmittel wurde fast vollständig im Vakuum abdestilliert und man erhielt 24.50 g eines farblosen Öls.

**N-(2-((Hexylcarbamoyl)oxy)ethyl)-N,N-dimethylhexadecan-1-aminium bromid (K-24)**

Zu einer Mischung von 29.4 g Hexylisocyanat und 0.05 g Desmorapid Z wurden bei 60 °C 20.6 g N,N-Dimethylethanol getropft und für 8 h bei dieser Temperatur gehalten. Nach Abkühlen auf Raumtemperatur wurden 50.0 g Amino-Urethan erhalten.

50.0 g Amino-Urethan wurden in 120 mL Acetonitril gelöst, 70.6 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 115.0 g eines farblosen klebrigen Harzes.

**$N^1$,$N^{16}$-Dihexadecyl-$N^1$,$N^1$,$N^{16}$,$N^{16}$,7,7,10-heptamethyl-4,13-dioxo-3,14-dioxa-5,12-diazahexadecan-1,16-diaminium dibromid (K-25)**

Zu einer Mischung von 27.0 g Vestanat TMDI (Produkt der EVONIK Deutschland) und 0.05 g Desmorapid Z wurden bei 60 °C 22.9 g N,N-Dimethylethanol getropft und für 8 h bei dieser Temperatur gehalten. Nach Abkühlen auf

Raumtemperatur wurden 50.0 g Amino-Urethan erhalten.

11.7 g Amino-Urethan wurden in 50 mL Acetonitril gelöst, 18.3 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 28.0 g eines farblosen klebrigen Harzes.

**N-(2-((((5-(((2-(Hexadecyldimethylammonio)ethoxy)carbonyl)amino)-1,3,3-trimethylcyclohexyl)methyl)carbamoyl)oxy)ethyl)-N,N-dimethylhexadecan-1-aminium dibromid (K-26)**

Zu einer Mischung von 27.7 g Desmodur I (Produkt der COVESTRO Deutschland) und 0.05 g Desmorapid Z wurden bei 60 °C 22.2 g N,N-Dimethylethanol getropft und für 8 h bei dieser Temperatur gehalten. Nach Abkühlen auf Raumtemperatur wurden 50.0 g Amino-Urethan erhalten.

11.9 g Amino-Urethan wurden in 50 mL Acetonitril gelöst, 18.1 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 29.3 g eines farblosen klebrigen Harzes.

**N,N'-(((((Methylenebis(cyclohexane-4,1-diyl))bis(azanediyl))bis(carbonyl))bis(oxy))-bis(ethan-2,1-diyl))bis(N,N-dimethylhexadecan-1-aminium) dibromid (K-27)**

Zu einer Mischung von 29.7 g Desmodur W (Produkt der COVESTRO Deutschland) und 0.05 g Desmorapid Z wurden bei 60 °C 20.2 g N,N-Dimethylethanol getropft und für 8 h bei dieser Temperatur gehalten. Nach Abkühlen auf Raumtemperatur wurden 50.0 g Amino-Urethan erhalten.

12.6 g Amino-Urethan wurden in 50 mL Acetonitril gelöst, 17.4 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 28.9 g eines farblosen klebrigen Harzes.

**N,N'-(((((4-Methyl-1,3-phenylene)bis(azanediyl))bis(carbonyl))bis(oxy))bis(ethan-2,1-diyl))bis(N,N-dimethylhexadecan-1-aminium) dibromid (K-28)**

Zu einer Mischung von 24.7 g Desmodur T80 (Produkt der COVESTRO Deutschland) und 0.05 g Desmorapid Z wurden bei 10 °C 25.3 g N,N-Dimethylethanol getropft und nach vollständiger Zugabe für 8 h bei 60°C gehalten. Nach Abkühlen auf Raumtemperatur wurden 50.0 g Amino-Urethan erhalten.

11.0 g Amino-Urethan wurden in 50 mL Acetonitril gelöst, 19.0 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 27.2 g eines farblosen klebrigen Harzes.

**N,N'-(((((Methylenebis(4,1-phenylene))bis(azanediyl))bis(carbonyl))bis(oxy))bis(ethan-2,1-diyl))bis(N,N-dimethylhexadecan-1-aminium) dibromid (K-29)**

Zu einer Mischung von 29.2 g Desmodur MDI (Produkt der COVESTRO Deutschland) und 0.05 g Desmorapid Z wurden bei 10 °C 20.8 g N,N-Dimethylethanol getropft und nach vollständiger Zugabe für 8 h bei 60°C gehalten. Nach Abkühlen auf Raumtemperatur wurden 50.0 g Amino-Urethan erhalten.

12.4 g Amino-Urethan wurden in 50 mL Acetonitril gelöst, 17.6 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 29.2 g eines farblosen klebrigen Harzes.

**N-(2-(2-((Hexylcarbamoyl)oxy)ethoxy)ethyl)-N,N-dimethylhexadecan-1-aminium bromid (K-30)**

Zu einer Mischung von 29.4 g Hexylisocyanat und 0.05 g Desmorapid Z wurden bei 60 °C 25.6 g 2-(2-Dimethylaminoethoxy)ethanol getropft und für 8 h bei dieser Temperatur gehalten. Nach Abkühlen auf Raumtemperatur wurden 50.0 g Amino-Urethan erhalten.

13.8 g Amino-Urethan wurden in 50 mL Acetonitril gelöst, 16.2 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 25.0 g farblosen Feststoff, Smp. 220-225 °C.

**$N^1$,$N^{22}$-Dihexadecyl-$N^1$,$N^1$,$N^{22}$,$N^{22}$,10,10,13-heptamethyl-7,16-dioxo-3,6,17,20-tetraoxa-8,15-diazadocosan-1,22-diaminium dibromid (K-31)**

Zu einer Mischung von 23.0 g Vestanat TMDI (Produkt der EVONIK Deutschland) und 0.05 g Desmorapid Z

wurden bei 60 °C 27.9 g 2-(2-Dimethylaminoethoxy)ethanol getropft und für 8 h bei dieser Temperatur gehalten. Nach Abkühlen auf Raumtemperatur wurden 50.0 g Amino-Urethan erhalten.

13.1 g Amino-Urethan wurden in 50 mL Acetonitril gelöst, 16.9 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 27.5 g eines farblosen klebrigen Harzes.

**N-(2-(2-(((3-(10,10-Dimethyl-3-oxo-4,7-dioxa-2,10-diazahexacosan-10-ium-1-yl)-3,5,5-trimethylcyclohexyl)carbamoyl)oxy)ethoxy)ethyl)-N,N-dimethylhexadecan-1-aminium dibromid (K-32)**

Zu einer Mischung von 22.7 g Desmodur I (Produkt der COVESTRO Deutschland) und 0.05 g Desmorapid Z wurden bei 60 °C 27.2 g 2-(2-Dimethylaminoethoxy)ethanol getropft und für 8 h bei dieser Temperatur gehalten. Nach Abkühlen auf Raumtemperatur wurden 50.0 g Amino-Urethan erhalten.

13.3 g Amino-Urethan wurden in 50 mL Acetonitril gelöst, 16.7 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 29.3 g eines farblosen klebrigen Harzes.

**N,N'-((((((Methylenebis(cyclohexan-4,1-diyl))bis(azanediyl))bis(carbonyl))bis(oxy))bis(ethan-2,1-diyl))bis(oxy))bis(ethan-2,1-diyl))bis(N,N-dimethylhexadecan-1-aminium) dibromid (K-33)**

Zu einer Mischung von 24.8 g Desmodur W (Produkt der COVESTRO Deutschland) und 0.05 g Desmorapid Z wurden bei 60 °C 25.2 g 2-(2-Dimethylaminoethoxy)ethanol getropft und für 8 h bei dieser Temperatur gehalten. Nach Abkühlen auf Raumtemperatur wurden 50.0 g Amino-Urethan erhalten.

13.9 g Amino-Urethan wurden in 50 mL Acetonitril gelöst, 16.1 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 25.9 g eines farblosen klebrigen Harzes.

**N,N'-((((((4-Methyl-1,3-phenylen)bis(azanediyl))bis-(carbonyl))bis(oxy))bis(ethan-2,1-diyl))bis(oxy))bis(ethan-2,1-diyl))bis(N,N-dimethylhexadecan-1-aminium) dibromid (K-34)**

Zu einer Mischung von 9.90 g Desmodur T80 (Produkt der COVESTRO Deutschland) und 0.05 g Desmorapid Z wurden bei 10 °C 15.1 g 2-(2-Dimethylaminoethoxy)ethanol getropft und nach vollständiger Zugabe für 8 h bei 60°C gehalten. Nach Abkühlen auf Raumtemperatur wurden 25.0 g Amino-Urethan erhalten.

12.6 g Amino-Urethan wurden in 50 mL Acetonitril gelöst, 17.4 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 23.1 g eines farblosen klebrigen Harzes.

**N,N'-((((((Methylen-bis(4,1-phenylene))bis(azanediyl))bis(carbonyl))bis(oxy))bis(ethan-2,1-diyl))bis(oxy))bis(ethan-2,1-diyl))bis(N,N-dimethylhexadecan-1-aminium) dibromid (K-35)**

Zu einer Mischung von 12.1 g Desmodur MDI (Produkt der COVESTRO Deutschland) und 0.05 g Desmorapid Z wurden bei 10 °C 12.9 g 2-(2-Dimethylaminoethoxy)ethanol getropft und nach vollständiger Zugabe für 8 h bei 60°C gehalten. Nach Abkühlen auf Raumtemperatur wurden 25.0 g Amino-Urethan erhalten.

13.8 g Amino-Urethan wurden in 50 mL Acetonitril gelöst, 16.2 g 1-Bromhexadecan wurden zugetropft und die Mischung wurde für 12 h am Rückfluss erhitzt. Der ausgefallene Feststoff wurde isoliert, mit kaltem Ether nachgewaschen, getrocknet und man erhielt 29.4 g eines farblosen klebrigen Harzes.

**Beispiele:**

Sofern nicht abweichend vermerkt beziehen sich alle Prozentangaben auf Gewichtsprozent.

**Herstellungsvorschrift a) für Triaryl-organoborate mit Kationen der Wertigkeit n=1**

10.0 mmol des angegebenen Boronsäureesters (I; $R^1$), 30.0 mmol Magnesiumspäne und 10.0 mmol des Salzes (II) wurden in einer Mischung aus 3.40 g wasserfreiem Toluol und 0.73 g wasserfreiem Tetrahydrofuran in einem trockenen Vierhalskolben mit mechanischem Rührer, Thermometer, Metallkühler und Tropftrichter mit Druckausgleich unter Stickstoffatmosphäre suspendiert und für 30 min intensiv gerührt. Zu dieser Lösung wurde zunächst unverdünnt der entsprechende Halogenaromat (III, $R^4$) gegeben, bis einsetzende Exothermie den Reaktionsstart signalisiert. Die restliche Menge von insgesamt 30.6 mmol Halogenaromat wurden mit 10.0 g wasserfreiem Toluol und 10.0 g wasserfreiem Tetrahydrofuran verdünnt und so zugetropft, dass die Innentemperatur 45 °C nicht überschreitet. Nach vollständiger Zugabe wurde das Reaktionsgemisch für 1 h oder bis zur vollständigen Auflösung des Magnesiums am Rückfluss erhitzt. Die erhaltene Suspension wurde auf 50 g Eis-Wasser ausgetragen, der erhaltene Feststoff wird isoliert, mit kaltem Wasser nachgewaschen und im Vakuum getrocknet. Die Beispiele 15, 18, 20 und 27 sowie 46 - 52 wurden mit 100 mL Ethylacetat aus der wässrigen Phase extrahiert, das Lösungsmittel wurde im Vakuum abdestilliert und die erhaltenen Öle isoliert. Die isolierte Ausbeute betrug für nach diesem Verfahren hergestelltes Beispiel 1 2.87 g (72 % d. Theorie), wogegen nach DE 198 50 139 A1 hergestelltes Beispiel 1 in einer Ausbeute von 47 % der Theorie erhalten wurde.

Die Zusammensetzung der Beispiele 1 - 76 und ausgewählte physikalische Eigenschaften sind in Tabelle 2 zusammengefasst.

Tabelle 2: Übersicht der Verbindungen der Formel (IV) mit n=1

| Beispiel | Einsatzstoffe gemäß Herstellungsvorschrift a) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [δ/ppm] | Smp. [°C] |
| 1 | Ethylboronsäure-Pinacolester | 1-Brom-4-Fluorbenzol | K-1 | Ethyl | 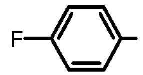 | -10.2 | 101-105 |
| 2 | Ethylboronsäure-Pinacolester | 1-Brom-4-Fluorbenzol | K-2 | Ethyl | 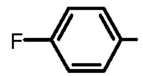 | -10.3 | 144-149 |
| 3 | Ethylboronsäu - re-Pinacolester | 1-Brom-3-chlor-4-methylbenzol | K-1 | Ethyl | 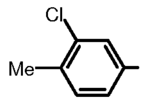 | -10.3 | 88-91 |
| 4 | Ethylboronsäure-Pinacolester | 1-Brom-3-chlor-4-methylbenzol | K-2 | Ethyl | 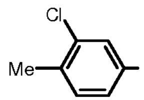 | -10.2 | 120-122 |
| 5 | Isopropylboronsäure-Pinacolester | 1-Brom-4-Fluorbenzol | K-2 | i-Propyl | 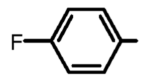 | -10.3 | 134-138 |
| 6 | Isopropylboronsäure-Pinacolester | 1-Brom-3-chlor-4-methylbenzol | K-2 | i-Propyl | 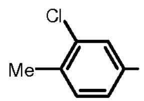 | -8.3 | 115-118 |
| 7 | 2-Isopropenylboronsäure-Pinacolester | 1-Brom-3-chlor-4-methylbenzol | K-2 | 2-Propenyl | 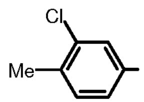 | -8.3 | amorph |
| 8 | Diisopropylallylboronate | 1-Brom-3-chlor-4-methylbenzol | K-1 | Allyl | 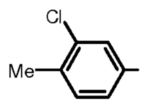 | -10.6 | amorph |
| 9 | Diisopropylallylboronate | 1-Brom-3-chlor-4-methylbenzol | K-2 | Allyl | 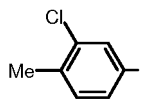 | -10.6 | 84-88 |

(fortgesetzt)

| Beispiel | Einsatzstoffe gemäß Herstellungsvorschrift a) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [δ/ppm] | Smp. [°C] |
| 10 | (1,3,2-Dioxaborinan-2-yl)cyclohexane | 1-Brom-4-Fluorbenzol | K-1 | Cyclohexyl | 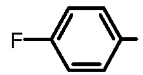 | -10.4 | 59-60 |
| 11 | (1,3,2-Dioxaborinan-2-yl)cyclohexane | 1-Brom-4-Fluorbenzol | K-2 | Cyclohexyl | 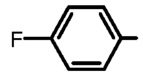 | -10.3 | 166-168 |
| 12 | (1,3,2-Dioxaborinan-2-yl)cyclohexane | 1-Brom-3-chlor-4-methylbenzol | K-2 | Cyclohexyl | 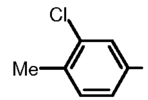 | -8.7 | 175-177 |
| 13 | 2-Hexyl-1,3,2-Dioxaborinane | 1-Brom-4-Fluorbenzol | K-2 | n-Hexyl | 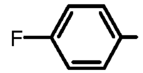 | -10.3 | 59-60 |
| 14 | 1-Dodecylboronsäure-Pinacolester | 1-Brom-4-Fluorbenzol | K-2 | n-Dodecyl | 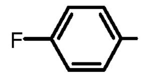 | -10.4 | Amorph |
| 15 | 2-Octadecyl-4,4,5,5-tetramethyl-1,3,2-Dioxaborolane | 1-Brom-4-Fluorbenzol | K-2 | n-Octadecyl | 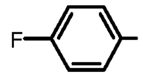 | -10.2 | Öl |
| 16 | 2-Hexyl-1,3,2-Dioxaborinane | 1-Brom-4-chlorbenzol | K-2 | n-Hexyl | 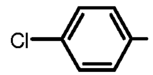 | -10.4 | 80-81 |
| 17 | 1-Dodecylboronsäure-Pinacolester | 1-Brom-4-chlorbenzol | K-2 | n-Dodecyl | 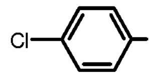 | -10.3 | Amorph |
| 18 | 2-Octadecyl-4,4,5,5-tetramethyl-1,3,2-Dioxaborolane | 1-Brom-4-chlorbenzol | K-2 | n-Octadecyl | 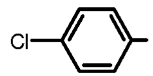 | -10.4 | Öl |
| 19 | 2-Hexyl-1,3,2-Dioxaborinane | 1-Brom-4-trifluormethylbenzol | K-2 | n-Hexyl | 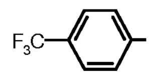 | -9.8 | 80-82 |

(fortgesetzt)

| Beispiel | Einsatzstoffe gemäß Herstellungsvorschrift a) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [$\delta$/ppm] | Smp. [°C] |
| 20 | 2-Octadecyl-4,4,5,5-tetramethyl-1,3,2-Dioxaborolane | 1-Brom-3-chlor-4-methylbenzol | K-2 | n-Octadecyl | 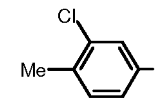 | -10.2 | Öl |
| 21 | 3-Phenyl-1-propylboronsäure-Pinacolester | 1-Brom-4-chlorbenzol | K-2 | 3-Phenylpropyl | 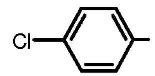 | -10.3 | 101-105 |
| 22 | 3-Phenyl-1-propylboronsäure-Pinacolester | 1-Brom-4-methoxybenzol | K-2 | 3-Phenylpropyl | 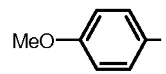 | -10.3 | 116-118 |
| 23 | 3-Phenyl-1-propylboronsäure-Pinacolester | 1-Brom-3,4,5-trifluorbenzol | K-2 | 3-Phenylpropyl | 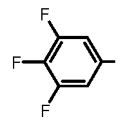 | -10.1 | 78-86 |
| 24 | 3-Phenyl-1-propylboronsäure-Pinacolester | 1-Brom-3-methyl-4-fluorbenzol | K-2 | 3-Phenylpropyl | 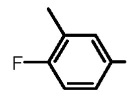 | -13.6 | 85-87 |
| 25 | 3-Phenyl-1-propylboronsäure-Pinacolester | 1-Brom-4-trifluormethoxybenzol | K-2 | 3-Phenylpropyl | 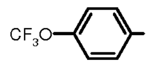 | -10.2 | 101-108 |
| 26 | 2-Hexyl-1,3,2-Dioxaborinane | 1-Brom-3-chlor-4-methylbenzol | K-3 | n-Hexyl | 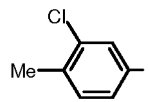 | -10.6 | amorph |
| 27 | 2-Hexyl-1,3,2-Dioxaborinane | 1-Brom-3-chlor-4-methylbenzol | K-4 | n-Hexyl | 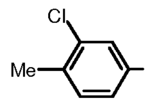 | -10.6 | Öl |
| 28 | 2-Hexyl-1,3,2-Dioxaborinane | 1-Brom-3-chlor-4-methylbenzol | K-5 | n-Hexyl | 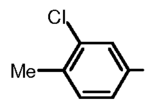 | -10.6 | amorph |

(fortgesetzt)

| Beispiel | Einsatzstoffe gemäß Herstellungsvorschrift a) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [$\delta$/ppm] | Smp. [°C] |
| 29 | 2-Hexyl-1,3,2-Dioxaborinane | 1-Brom-3-chlor-4-methylbenzol | K-6 | n-Hexyl | 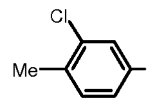 | -10.6 | amorph |
| 30 | 2-Hexyl-1,3,2-Dioxaborinane | 1-Brom-3-chlor-4-methylbenzol | K-7 | n-Hexyl | 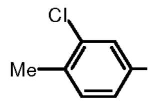 | -10.6 | amorph |
| 31 | 2-Hexyl-1,3,2-Dioxaborinane | 1-Brom-3-chlor-4-methylbenzol | K-8 | n-Hexyl | 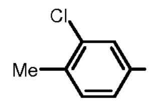 | -10.6 | amorph |
| 32 | 2-Hexyl-1,3,2-Dioxaborinane | 1-Brom-3-chlor-4-methylbenzol | K-9 | n-Hexyl | 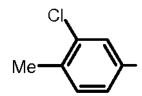 | -10.6 | amorph |
| 33 | 2-Hexyl-1,3,2-Dioxaborinane | 1-Brom-3-chlor-4-methylbenzol | K-10 | n-Hexyl | 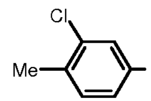 | -10.6 | amorph |
| 34 | 2-Hexyl-1,3,2-Dioxaborinane | 1-Brom-3-chlor-4-methylbenzol | K-11 | n-Hexyl | 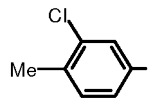 | -10.6 | amorph |
| 35 | 2-Hexyl-1,3,2-Dioxaborinane | 1-Brom-3-chlor-4-methylbenzol | K-12 | n-Hexyl | 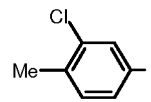 | -10.6 | amorph |
| 36 | 2-Hexyl-1,3,2-Dioxaborinane | 1-Brom-3-chlor-4-methylbenzol | K-13 | n-Hexyl | 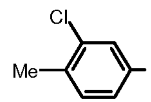 | -10.6 | amorph |

(fortgesetzt)

| Beispiel | Einsatzstoffe gemäß Herstellungsvorschrift a) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [δ/ppm] | Smp. [°C] |
| 37 | 2-Hexyl-1,3,2-Dioxaborinane | 1-Brom-3-chlor-4-methylbenzol | K-16 | n-Hexyl | 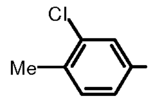 | -10.6 | amorph |
| 38 | 2-Hexyl-1,3,2-Dioxaborinane | 1-Brom-3-chlor-4-methylbenzol | K-17 | n-Hexyl | 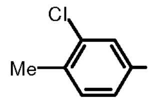 | -10.6 | amorph |
| 39 | 2-Hexyl-1,3,2-Dioxaborinane | 1-Brom-3-chlor-4-methylbenzol | K-19 | n-Hexyl | 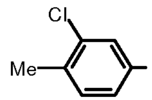 | -10.6 | amorph |
| 40 | 2-Hexyl-1,3,2-Dioxaborinane | 1-Brom-3-chlor-4-methylbenzol | K-20 | n-Hexyl | 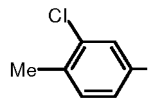 | -10.6 | amorph |
| 41 | 2-Hexyl-1,3,2-Dioxaborinane | 1-Brom-3-chlor-4-methylbenzol | K-21 | n-Hexyl | 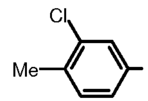 | -10.6 | amorph |
| 42 | 2-Hexyl-1,3,2-Dioxaborinane | 1-Brom-3-chlor-4-methylbenzol | K-22 | n-Hexyl | 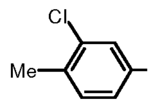 | -10.6 | amorph |
| 43 | 2-Hexyl-1,3,2-Dioxaborinane | 1-Brom-3-chlor-4-methylbenzol | K-23 | n-Hexyl | 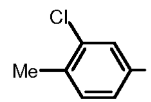 | -10.6 | amorph |
| 44 | 2-Hexyl-1,3,2-Dioxaborinane | 1-Brom-3-chlor-4-methylbenzol | K-24 | n-Hexyl | 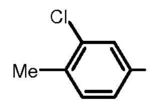 | -10.6 | amorph |

(fortgesetzt)

| Beispiel | Einsatzstoffe gemäß Herstellungsvorschrift a) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [δ/ppm] | Smp. [°C] |
| 45 | 2-Hexyl-1,3,2-Dioxaborinane | 1-Brom-3-chlor-4-methylbenzol | K-30 | n-Hexyl | 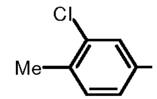 | -10.6 | amorph |
| 46 | 1-Dodecylboronsäure-Pinacolester | 1-Brom-4-chlorbenzol | K-2 | n-Dodecyl | 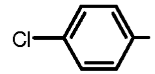 | -10.3 | Öl |
| 47 | 1-Dodecylboronsäure-Pinacolester | 1-Brom-4-methoxybenzol | K-2 | n-Dodecyl | 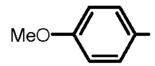 | -10.4 | Öl |
| 48 | 1-Dodecylboronsäure-Pinacolester | 1-Brom-3,4,5-trifluorbenzol | K-2 | n-Dodecyl | 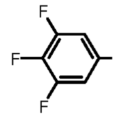 | -10.1 | Öl |
| 49 | 1-Dodecylboronsäure-Pinacolester | 1-Brom-3-methyl-4-fluorbenzol | K-2 | n-Dodecyl | 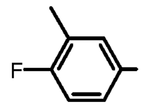 | -10.6 | Öl |
| 50 | 1-Dodecylboronsäure-Pinacolester | 1-Brom-4-trifluormethoxybenzol | K-2 | n-Dodecyl | 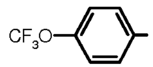 | -10.8 | Öl |
| 51 | 2-Hexyl-1,3,2-Dioxaborinane | 1-Brom-3-chlor-4-methylbenzol | K-100 | n-Hexyl | 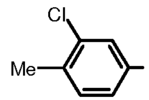 | -10.6 | Öl |
| 52 | 2-Hexyl-1,3,2-Dioxaborinane | 1-Brom-3-chlor-4-methylbenzol | K-101 | n-Hexyl | 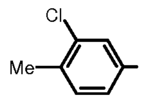 | -10.6 | Öl |
| 53 | 2-Hexyl- 1,3,2-Dioxaborinane | 1-Brom-3-chlor-4-methylbenzol | K-102 | n-Hexyl | 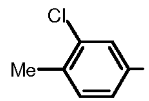 | -10.6 | Zers. |

(fortgesetzt)

| Beispiel | Einsatzstoffe gemäß Herstellungsvorschrift a) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [δ/ppm] | Smp. [°C] |
| 54 | 2-Hexyl-1,3,2-Dioxaborinane | 1-Brom-3-chlor-4-methylbenzol | K-103 | n-Hexyl | 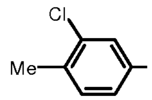 | -10.6 | 102-105 |
| 55 | 2-Hexyl-1,3,2-Dioxaborinane | 1-Brom-3-chlor-4-methylbenzol | K-104 | n-Hexyl | 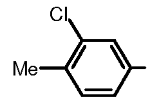 | -10.6 | 134-138 |
| 56 | 2-Hexyl-1,3,2-Dioxaborinane | 1-Brom-3-chlor-4-methylbenzol | K-105 | n-Hexyl | 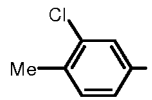 | -10.6 | 145-146 |
| 57 | 2-Hexyl-1,3,2-Dioxaborinane | 1-Brom-3-chlor-4-methylbenzol | K-106 | n-Hexyl | 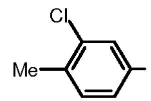 | -10.6 | 155-159 |
| 58 | 2-Hexyl-1,3,2-Dioxaborinane | 1-Brom-3-chlor-4-methylbenzol | K-200 | n-Hexyl | 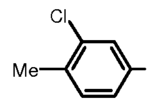 | -10.4 | amorph |
| 59 | 2-Hexyl-1,3,2-Dioxaborinane | 1-Brom-3-chlor-4-methylbenzol | K-201 | n-Hexyl | 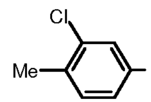 | -10.4 | amorph |
| 60 | 2-Hexyl-1,3,2-Dioxaborinane | 1-Brom-3-chlor-4-methylbenzol | K-202 | n-Hexyl | 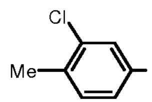 | -10.4 | amorph |
| 61 | 2-Hexyl-1,3,2-Dioxaborinane | 1-Brom-3-chlor-4-methylbenzol | K-300 | n-Hexyl | 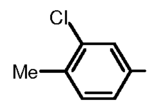 | -10.6 | amorph |

(fortgesetzt)

| Beispiel | Einsatzstoffe gemäß Herstellungsvorschrift a) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [δ/ppm] | Smp. [°C] |
| 62 | 2-Hexyl-1,3,2-Dioxaborinane | 1-Brom-3-chlor-4-methylbenzol | K-303 | n-Hexyl | 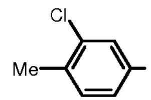 | -10.6 | amorph |
| 63 | 2-Hexyl-1,3,2-Dioxaborinane | 1-Brom-3-chlor-4-methylbenzol | K-303 | n-Hexyl | 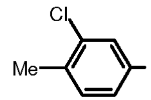 | -10.6 | amorph |
| 64 | 2-Hexyl-1,3,2-Dioxaborinane | 1-Brom-3-chlor-4-methylbenzol | K-304 | n-Hexyl | 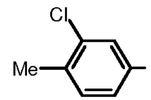 | -10.6 | amorph |
| 65 | 2-Hexyl-1,3,2-Dioxaborinane | 1-Brom-3-chlor-4-methylbenzol | K-305 | n-Hexyl | 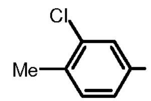 | -10.6 | amorph |
| 66 | 2-Hexyl-1,3,2-Dioxaborinane | 1-Brom-3-chlor-4-methylbenzol | K-400 | n-Hexyl | 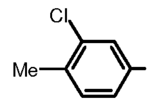 | -10.5 | amorph |
| 67 | 2-Hexyl-1,3,2-Dioxaborinane | 1-Brom-3-chlor-4-methylbenzol | K-401 | n-Hexyl | 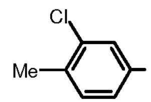 | -10.5 | amorph |
| 68 | 2-Hexyl-1,3,2-Dioxaborinane | 1-Brom-3-chlor-4-methylbenzol | K-402 | n-Hexyl | 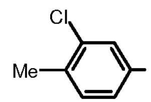 | -10.5 | amorph |
| 69 | 2-Hexyl-1,3,2-Dioxaborinane | 1-Brom-3-chlor-4-methylbenzol | K-403 | n-Hexyl | 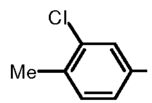 | -10.5 | amorph |

(fortgesetzt)

| Beispiel | Einsatzstoffe gemäß Herstellungsvorschrift a) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [$\delta$/ppm] | Smp. [°C] |
| 70 | 2-Hexyl-1,3,2-Dioxaborinan | 1-Brom-3-chlor-4-methylbenzol | K-37 | n-Hexyl | 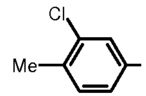 | -10.5 | amorph |
| 71 | 2-Hexyl-1,3,2-Dioxaborinan | 1-Brom-3-chlor-4-methylbenzol | K-38 | n-Hexyl | 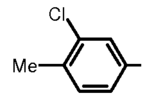 | -10.5 | amorph |
| 72 | 2-Hexyl-1,3,2-Dioxaborinan | 1-Brom-3-chlor-4-methylbenzol | K-39 | n-Hexyl | 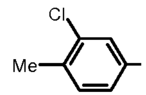 | -10.5 | amorph |
| 73 | 2-Hexyl-1,3,2-Dioxaborinan | 1-Brom-3-chlor-4-methylbenzol | K-40 | n-Hexyl | 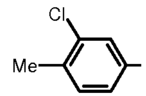 | -10.5 | amorph |
| 74 | 2-Hexyl-1,3,2-Dioxaborinan | 1-Brom-3-chlor-4-methylbenzol | K-41 | n-Hexyl | 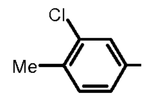 | -10.5 | amorph |
| 75 | 2-Hexyl-1,3,2-Dioxaborinan | 1-Brom-3-chlor-4-methylbenzol | K-42 | n-Hexyl | 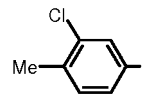 | -10.5 | amorph |
| 76 | 2-Hexyl-1,3,2-Dioxaborinan | 1-Brom-3-chlor-4-methylbenzol | K-43 | n-Hexyl | 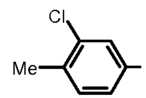 | -10.5 | amorph |

**Herstellungsvorschrift b) für Triaryl-organoborate mit Kationen der Wertigkeit n=2**

10.0 mmol des angegebenen Boronsäureesters (I; $R^1$), 30.0 mmol Magnesiumspäne und 5.0 mmol des Salzes (II) werden in einer Mischung aus 3.40 g wasserfreiem Toluol und 0.73 g wasserfreiem Tetrahydrofuran in einem trockenen Vierhalskolben unter Stickstoffatmosphäre mit mechanischem Rührer, Thermometer, Metallkühler und Tropftrichter mit Druckausgleich suspendiert und für 30 min intensiv gerührt. Zu dieser Lösung wird zunächst unverdünnt der entsprechende Halogenaromat (III, $R^4$) gegeben, bis einsetzende Exothermie den Reaktionsstart signalisiert. Die restliche Menge von insgesamt 30.6 mmol Halogenaromat werden mit 10.0 g wasserfreiem Toluol und 10.0 g wasserfreiem Tetrahydrofuran verdünnt und so zugetropft, dass die Innentemperatur 45 °C nicht überschreitet. Nach vollständiger Zugabe wird das Reaktionsgemisch für 1 h oder bis zur vollständigen Auflösung des Magnesiums am Rückfluss erhitzt. Die erhaltene Suspension wird auf 50 g Eis-Wasser ausgetragen, der erhaltene Feststoff wird isoliert, mit kaltem Wasser nachgewaschen und im Vakuum getrocknet.

Die Zusammensetzung der Beispiele 77 - 91 und ausgewählte physikalische Eigenschaften sind in Tabelle 3 zusammengefasst.

Tabelle 3: Übersicht der Verbindungen der Formel (IV) mit n=2

| Beispiel | Einsatzstoffe gemäß Herstellungsvorschrift b) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [δ/ppm] | Smp. [°C] |
| 77 | 2-Hexyl-1,3,2-Dioxaborinane | 1-Brom-3-chlor-4-methylbenzol | K-14 | n-Hexyl | 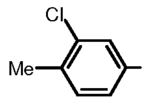 | -10.6 | amorph |
| 78 | 2-Hexyl-1,3,2-Dioxaborinane | 1-Brom-3-chlor-4-methylbenzol | K-15 | n-Hexyl | 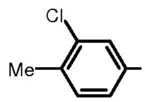 | -10.6 | amorph |
| 79 | 2-Hexyl-1,3,2-Dioxaborinane | 1-Brom-3-chlor-4-methylbenzol | K-18 | n-Hexyl | 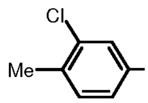 | -10.6 | amorph |
| 80 | 2-Hexyl-1,3,2-Dioxaborinane | 1-Brom-3-chlor-4-methylbenzol | K-25 | n-Hexyl | 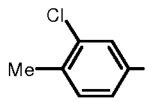 | -10.6 | amorph |
| 81 | 2-Hexyl-1,3,2-Dioxaborinane | 1-Brom-3-chlor-4-methylbenzol | K-26 | n-Hexyl | 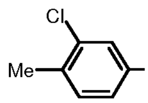 | -10.6 | amorph |
| 82 | 2-Hexyl-1,3,2-Dioxaborinane | 1-Brom-3-chlor-4-methylbenzol | K-27 | n-Hexyl | 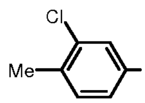 | -10.6 | amorph |
| 83 | 2-Hexyl-1,3,2-Dioxaborinane | 1-Brom-3-chlor-4-methylbenzol | K-28 | n-Hexyl | 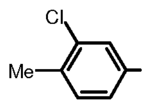 | -10.6 | amorph |
| 84 | 2-Hexyl-1,3,2-Dioxaborinane | 1-Brom-3-chlor-4-methylbenzol | K-29 | n-Hexyl | 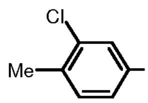 | -10.6 | amorph |
| 85 | 2-Hexyl-1,3,2-Dioxaborinane | 1-Brom-3-chlor-4-methylbenzol | K-31 | n-Hexyl | 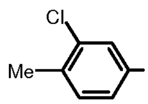 | -10.6 | amorph |

(fortgesetzt)

| Beispiel | Einsatzstoffe gemäß Herstellungsvorschrift b) | | | Substituenten gemäß Formel (IV) $K^+$ und Charakterisierung | | | |
|---|---|---|---|---|---|---|---|
| | Boronsäureester (I) | Halogenaromat (III) | Salz (II) | $R^1$ | $R^4$ | $^{11}B$ [δ/ppm] | Smp. [°C] |
| 86 | 2-Hexyl-1,3,2-Dioxaborinane | 1-Brom-3-chlor-4-methylbenzol | K-32 | n-Hexyl | 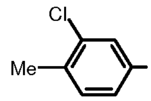 | -10.6 | amorph |
| 87 | 2-Hexyl-1,3,2-Dioxaborinane | 1-Brom-3-chlor-4-methylbenzol | K-33 | n-Hexyl | 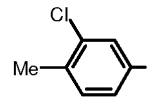 | -10.6 | amorph |
| 88 | 2-Hexyl-1,3,2-Dioxaborinane | 1-Brom-3-chlor-4-methylbenzol | K-34 | n-Hexyl | 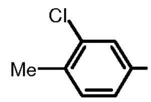 | -10.6 | amorph |
| 89 | 2-Hexyl-1,3,2-Dioxaborinane | 1-Brom-3-chlor-4-methylbenzol | K-35 | n-Hexyl | 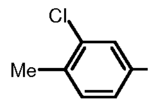 | -10.6 | amorph |
| 90 | 2-Hexyl-1,3,2-Dioxaborinane | 1-Brom-3-chlor-4-methylbenzol | K-203 | n-Hexyl | 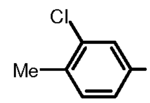 | -10.4 | amorph |
| 91 | 2-Hexyl-1,3,2-Dioxaborinane | 1-Brom-3-chlor-4-methylbenzol | K-301 | n-Hexyl | 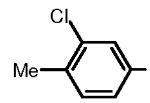 | -10.6 | amorph |

**Beispiel 92 (Kation der Wertigkeit n=3):**

1.70 g 2-Hexyl-1,3,2-Dioxaborinane (10.0 mmol; I; $R^1$ = n-Hexyl), 0.73 g (30.0 mmol) Magnesiumspäne und 2.25 g $N^1$,$N^1$,$N^1$,$N^3$,$N^3$,$N^3$,$N^5$,$N^5$,$N^5$-Nonamethyl-1,3,5-benzentrimethanaminium triiodid (3.33 mmol; K-36; II) werden in einer Mischung aus 3.40 g wasserfreiem Toluol und 0.73 g wasserfreiem Tetrahydrofuran in einem trockenen Vierhalskolben mit mechanischem Rührer, Thermometer, Metallkühler und Tropftrichter mit Druckausgleich unter Stickstoffatmosphäre suspendiert und für 30 min intensiv gerührt. Zu dieser Lösung wird zunächst unverdünntes 1-Brom-3-chlor-4-methylbenzol (III, $R^4$) gegeben, bis einsetzende Exothermie den Reaktionsstart signalisiert. Die restliche Menge von insgesamt 6.29 g (30.6 mmol) 1-Brom-3-chlor-4-methylbenzol werden mit 10.0 g wasserfreiem Toluol und 10.0 g wasserfreiem Tetrahydrofuran verdünnt und so zugetropft, dass die Innentemperatur 45 °C nicht überschreitet. Nach vollständiger Zugabe wird das Reaktionsgemisch für 1 h oder bis zur vollständigen Auflösung des Magnesiums am Rückfluss erhitzt. Die erhaltene Suspension wird auf 50 g Eis-Wasser ausgetragen, der erhaltene Feststoff wird isoliert, mit kaltem Wasser nachgewaschen und im Vakuum getrocknet.

$^{11}B$-NMR: [δ/ppm] -10.6.

**Herstellung der Medien zur Bestimmung der holographischen Eigenschaften**

**Beispiel-Medium I**

3.38 g der Polyol-Komponente 1 wurden mit 0.010 g Beispiel 1, 2.00 g Urethanacrylat 1, 2.00 g Urethanacrylat 2, 1.50 g Additiv 1, 0.10 g Triaryl-organoborat 1 gemäß Formel (IV), 0.010 g Farbstoff 1 und 0.35 g N-Ethylpyrrolidon bei 60 °C gemischt, so dass eine klare Lösung erhalten wurde. Anschließend wurde auf 30 °C abgekühlt, 0.65 g Desmodur® N3900 zugegeben und erneut gemischt. Schließlich wurden 0.01 g Fomrez UL 28 zugegeben und erneut kurz gemischt. Die erhaltene, flüssige Masse wurde dann auf eine Glasplatte gegeben und dort mit einer zweiten Glasplatte abgedeckt. Dieser Probenkörper wurde 12 Stunden bei Raumtemperatur liegen gelassen und gehärtet.

**Beispiel-Medium II-XX**

Es wurde gearbeitet wie im Beispiel Medium I, aber unter Verwendung von 0.10 g des angegebenen Triaryl-organoborates gemäß Formel (IV) anstatt 0.10 g Triaryl-organoborat 1. Die holographischen Eigenschaften der Beispiel-Medien I - XX sind in Tabelle 4 zusammengefasst.

Tabelle 4: Übersicht der holographischen Eigenschaften der Beispiel-Medien I-XX

| Beispiel-Medium | Beispiel gemäß Formel (IV) | $\Delta n$ |
|---|---|---|
| I | 1 | 0.033 |
| II | 13 | 0.029 |
| III | 16 | 0.029 |
| IV | 17 | 0.034 |
| V | 18 | 0.033 |
| VI | 19 | 0.032 |
| VII | 20 | 0.037 |
| VIII | 24 | 0.033 |
| IX | 29 | 0.034 |
| X | 45 | 0.039 |
| XI | 51 | 0.035 |
| XII | 52 | 0.034 |
| XIII | 57 | 0.037 |
| XIV | 66 | 0.038 |
| XV | 77 | 0.037 |
| XVI | 79 | 0.040 |
| XVII | 81 | 0.040 |
| XVIII | 85 | 0.037 |
| XIX | 86 | 0.035 |
| XX | 88 | 0.037 |

Die gefundenen Werte für die Beispiel-Medien I bis XX zeigen, dass die in den Photopolymer-Formulierungen eingesetzten Verbindungen der Formel (IV) für die Verwendung in holographischen Medien sehr gut geeignet sind.

**Patentansprüche**

**1.** Verfahren zur Herstellung von Triaryl-organoboraten der Formel $1/n\ K^{n+}R^4_3B^-\text{-}R^1$ (IV), wobei ein Äquivalent eines Organo-Boronsäureester der Formel $B\text{-}R^1(OR^2)(OR^3)$ (I) mit 1/n Äquivalenten Salz $K^{n+}\ nX^-$ (II) und 3 Äquivalenten Metall M in einem Lösungsmittel oder einem Lösungsmittelgemisch S vorgelegt, 3 Äquivalente eines Halogen-Aromaten $R^4$-Y (III) zugegeben werden, Wasser und gegebenenfalls ein zweites organisches Lösungsmittel S zugegeben und die Verbindung $1/n\ K^{n+}R^4_3B^-\text{-}R^1$ (IV) mit der organischen Phase abgetrennt wird, und

$R^1$ für einen gegebenenfalls mit Hydroxy und / oder Alkoxy und / oder Acyloxy und / oder Halogen substituierten $C_1$- bis $C_{22}$-Alkyl-, $C_3$- bis $C_{22}$-Alkenyl-, $C_3$- bis $C_{22}$-Alkinyl-, $C_5$- bis $C_7$-Cycloalkyl- oder $C_7$- bis $C_{13}$-Aralkylrest steht und
$R^2$ und $R^3$ unabhängig voneinander für einen gegebenenfalls verzweigten $C_1$- bis $C_{22}$-Alkyl-Rest oder einen gegebenenfalls durch Alkyl substituierten $C_3$- bis $C_7$-Cycloalkyl-Rest stehen oder $R^2$ und $R^3$ gemeinsam eine

2-8 gliedrige gegebenenfalls durch Alkyl substituierte und / oder durch Sauerstoff-Atome unterbrochene Kohlenstoff-Brücke bilden und
$R^4$ für einen $C_6$- bis $C_{10}$-Arylrest steht, der gegebenenfalls durch mindestens einen Rest der Auswahl Halogen, $C_1$-bis $C_4$-Alkyl, Trifluormethyl, $C_1$-bis $C_4$-Alkoxy, Trifluormethoxy, Phenyl oder Phenoxy substituiert ist und
K für ein beliebig substituiertes Organokation der Wertigkeit n auf Basis von Stickstoff, Phosphor, Sauerstoff, Schwefel, und / oder Iod steht und
M für ein Metall beliebig ausgewählt aus den Alkalimetallen, Magnesium, Cal-cium oder Aluminium und
X für ein Halogenid oder Alkoxyd oder Alkylsulfid und
Y für Iod oder Brom oder Chlor und
n für 1, 2 oder 3 und
S für ein aprotisches organisches Lösungsmittel oder ein Gemisch aprotischer Lösungsmittel steht.

**2.** Verfahren zur Herstellung von Verbindungen 1/n $1/n\ K^{n+}R^4_3B^-\text{-}R^1$ der Formel (IV) gemäß Anspruch 1 umfassend die Schritte

i) das Vorlegen von einem Äquivalent Organo-Boronsäureester B-$R^1$($OR^2$)($OR^3$) (I) mit 1/n Äquivalenten Salz $K^{n+}$ $nX^-$ (II) und 3 Äquivalenten Metall M in einem Lösungsmittel oder einem Lösungsmittelgemisch S,
ii) die Zugabe von 3 Äquivalenten eines Halogen-Aromaten $R^4$-Y (III), wodurch
iii) ein *in situ* erzeugtes metallorganisches Reagenz mit den vorgelegten Substanzen (I) und (II) zum $1/n\ K^{n+}R^4_3B^-\text{-}R^1$ (IV) reagiert.

**3.** Verfahren gemäß Ansprüchen 1 oder 2, **dadurch gekennzeichnet, dass** es sich bei den Organokationen K der Wertigkeit n auf Basis von Stickstoff um Ammoniumionen, Pyridiniumionen und Imidazoliumionen, die gegebenenfalls in einer oder mehreren Seitenketten weitere funktionelle Gruppen wie Ether, Ester, Amide und / oder Carbamate tragen und die auch in oligomerer oder polymerer oder verbrückender Form vorliegen können, handelt.

**4.** Verfahren gemäß Ansprüchen 1 oder 2, **dadurch gekennzeichnet, dass** es sich bei den Organokationen K der Wertigkeit n auf Basis von Phosphor um beliebig substituierte Tetraalkyl-Phosphonium-, Trialkyl-Aryl-Phosphonium-, Dialkyl-diaryl-Phosphonium-, Alkyl-Triaryl-Phosphonium- oder Tetraaryl-Phosphonium-Salze, die gegebenenfalls in einer oder mehreren Seitenketten weitere funktionelle Gruppen wie Carbonyle, Amide und / oder Carbamate tragen und die auch in oligomerer oder polymerer oder verbrückender Form vorliegen können, handelt.

**5.** Verfahren gemäß Ansprüchen 1 oder 2, **dadurch gekennzeichnet, dass** es sich bei den Organokation K der Wertigkeit n auf Basis von Sauerstoff um beliebig substituiertes Pyrylium, auch in annellierter Form wie im Benzopyrylium, Flavylium Naphthoxanthenium oder polymere Kationen mit den genannten Substitutionsmustern handelt.

**6.** Verfahren gemäß Ansprüchen 1 oder 2, **dadurch gekennzeichnet, dass** es sich bei den Organokationen K der Wertigkeit n auf Basis von Schwefel um Onium-Verbindungen des Schwefels, die gleiche oder verschiedene gegebenenfalls substituierte $C_6$- bis $C_{12}$-Alkyl-, $C_6$- bis $C_{10}$-Aryl-, $C_7$- bis $C_{12}$-Arylalkyl- oder $C_5$- bis $C_6$-Cycloalkylreste tragen und / oder oligomere oder polymere wiederkehrende verbindende Einheiten zu Sulfoniumsalzen mit $1 \leq n \leq$ 3aufbauen sowie Thiopyrylium oder polymere Kationen mit den genannten Substitutionsmustern handelt.

**7.** Verfahren gemäß Ansprüchen 1 oder 2, **dadurch gekennzeichnet, dass** es sich bei den Organokationen K der Wertigkeit n auf Basis von Iod um Onium-Verbindungen des Iods, die gleiche oder verschiedene gegebenenfalls substituierte $C_4$- bis $C_{12}$-Alkyl-, $C_6$-bis $C_{10}$-Aryl-, $C_7$- bis $C_{12}$-Arylalkyl- oder $C_5$- bis $C_6$-Cycloalkylreste tragen und / oder oligomere oder polymere wiederkehrende verbindende Einheiten zu Iodoniumsalzen mit $1 \leq n \leq 3$ aufbauen oder um weitere polymere Kationen mit den genannten Substitutionsmustern handelt.

**8.** Verfahren gemäß einem der Ansprüche 1 bis 7, **dadurch gekennzeichnet, dass** $R^1$ für einen gegebenenfalls mit Hydroxy und / oder Alkoxy und / oder Acyloxy und / oder Halogen substituierten $C_2$- bis $C_{18}$-Alkyl-, $C_3$- bis $C_{18}$-Alkenyl-, $C_3$- bis $C_{18}$-Alkinyl-, $C_5$-bis $C_6$-Cycloalkyl- oder $C_7$- bis $C_{13}$-Aralkylrest steht.

**9.** Verfahren gemäß einem der Ansprüche 1 bis 8, **dadurch gekennzeichnet, dass** $R^2$ und $R^3$ unabhängig voneinander für einen gegebenenfalls verzweigten $C_2$- bis $C_{18}$-Alkyl-Rest stehen oder $R^2$ und $R^3$ bilden gemeinsam einen 4-7 gliedrigen gegebenenfalls substituierten Carbocylus.

**10.** Verfahren gemäß einem der Ansprüche 1 bis 9, **dadurch gekennzeichnet, dass** $R^4$ für einen $C_6$- bis $C_{10}$-Arylrest, der gegebenenfalls durch mindestens einen Rest der Auswahl Halogen, $C_1$-bis $C_4$-Alkyl, Trifluormethyl, $C_1$-bis $C_4$-Alkoxy, Trifluormethoxy, Phenyl und / oder Phenoxy substituiert ist, steht.

## Claims

**1.** Process for preparing triaryl organoborates of the formula $1/n\ K^{n+}R^4_3B^{-}-R^1$ (IV), where one equivalent of an organoboronic ester of the formula B-$R^1$ ($OR^2$) ($OR^3$) (I) is initially charged together with 1/n equivalents of salt $K^{n+}$ $nX^-$ (II) and 3 equivalents of metal M in a solvent or a solvent mixture S, 3 equivalents of a haloaromatic $R^4$-Y (III) are added, water and optionally a second organic solvent S is added and the compound $1/n\ K^{n+}\ R^4_3B^{-}-R^1$ (IV) is separated off with the organic phase, and

$R^1$ is an optionally hydroxyl- and/or alkoxy- and/or acyloxy- and/or halogen-substituted $C_1$- to $C_{22}$-alkyl, $C_3$- to $C_{22}$-alkenyl, $C_3$- to $C_{22}$-alkynyl, $C_5$-to $C_7$-cycloalkyl or $C_7$- to $C_{13}$-aralkyl radical and

$R^2$ and $R^3$ are independently an optionally branched $C_1$- to $C_{22}$-alkyl radical or an optionally alkylsubstituted $C_3$- to $C_7$-cycloalkyl radical or $R^2$ and $R^3$ together form a 2-8-membered carbon bridge which is optionally substituted by alkyl and/or interrupted by oxygen atoms and

$R^4$ is a $C_6$- to $C_{10}$-aryl radical optionally substituted by at least one radical selected from halogen, $C_1$-to $C_4$-alkyl, trifluoromethyl, $C_1$-to $C_4$-alkoxy, trifluoromethoxy, phenyl and phenoxy and

K is an organocation of valency n and having any substitution, based on nitrogen, phosphorus, oxygen, sulfur and/or iodine, and

M is any metal selected from the alkali metals, magnesium, calcium and aluminium and

X is a halide or alkoxide or alkyl sulfide and

Y is iodine or bromine or chlorine and

n is 1, 2 or 3 and

S is an aprotic organic solvent or a mixture of aprotic solvents.

**2.** Process for preparing compounds $1/n\ K^{n+}\ R^4_3B^{-}-R^1$ of the formula (IV) according to Claim 1, comprising the steps of

i) initially charging one equivalent of organoboronic ester B-$R^1$($OR^2$)($OR^3$) (I) together with 1/n equivalents of salt $K^{n+}$ $nX^-$ (II) and 3 equivalents of metal M in a solvent or a solvent mixture S,
ii)adding 3 equivalents of a haloaromatic $R^4$-Y (III), as a result of which
iii) an organometallic reagent generated *in situ* reacts with the initially charged substances (I) and (II) to give $1/n\ K^{n+}\ R^4_3B^{-}-R^1$ (IV).

**3.** Process according to Claim 1 or 2, **characterized in that** the organocations K of valency n based on nitrogen are ammonium ions, pyridinium ions and imidazolium ions, which optionally bear further functional groups such as ethers, esters, amides and/or carbamates in one or more side chains and which may also be in oligomeric or polymeric or bridging form.

**4.** Process according to Claim 1 or 2, **characterized in that** the organocations K of valency n based on phosphorus are tetraalkylphosphonium, trialkylarylphosphonium, dialkyldiarylphosphonium, alkyltriarylphosphonium or tetraarylphosphonium salts having any substitution, which optionally bear further functional groups such as carbonyls, amides and/or carbamates in one or more side chains and which may also be in oligomeric or polymeric or bridging form.

**5.** Process according to Claim 1 or 2, **characterized in that** the organocation K of valency n based on oxygen is pyrylium having any substitution, including in fused form as in benzopyrylium, flavylium, naphthoxanthenium or polymeric cations with the substitution patterns mentioned.

**6.** Process according to Claim 1 or 2, **characterized in that** the organocations K of valency n based on sulfur are onium compounds of sulfur that bear identical or different optionally substituted $C_6$- to $C_{12}$-alkyl, $C_6$- to $C_{10}$-aryl, $C_7$- to $C_{12}$-arylalkyl or $C_5$-to $C_6$-cycloalkyl radicals and/or that form oligomeric or polymeric repeating connecting units to give sulfonium salts with $1 \leq n \leq 3$, and thiopyrylium or polymeric cations with the substitution patterns mentioned.

7. Process according to Claim 1 or 2, **characterized in that** the organocations K of valency n based on iodine are onium compounds of iodine that bear identical or different optionally substituted $C_4$- to $C_{12}$-alkyl, $C_6$- to $C_{10}$-aryl, $C_7$- to $C_{12}$-arylalkyl or $C_5$-to $C_6$-cycloalkyl radicals and/or that form oligomeric or polymeric repeating connecting units to give iodonium salts with $1 \leq n \leq 3$, or are further polymeric cations with the substitution patterns mentioned.

8. Process according to any of Claims 1 to 7, **characterized in that** $R^1$ is an optionally hydroxyl- and/or alkoxy- and/or acyloxy- and/or halogen-substituted $C_2$- to $C_{18}$-alkyl, $C_3$- to $C_{18}$-alkenyl, $C_3$-to $C_{18}$-alkynyl, $C_5$- to $C_6$-cycloalkyl or $C_7$- to $C_{13}$-aralkyl radical.

9. Process according to any of Claims 1 to 8, **characterized in that** $R^2$ and $R^3$ are independently an optionally branched $C_2$- to $C_{18}$-alkyl radical or $R^2$ and $R^3$ together form a 4-7-membered, optionally substituted carbocycle.

10. Process according to any of Claims 1 to 9, **characterized in that** $R^4$ is a $C_6$- to $C_{10}$-aryl radical optionally substituted by at least one radical selected from halogen, $C_1$-to $C_4$-alkyl, trifluoromethyl, $C_1$-to $C_4$-alkoxy, trifluoromethoxy, phenyl and/or phenoxy.

## Revendications

1. Procédé pour la préparation de triaryl-organoborates de formule 1/n $K^{n+}R^4{}_3B^-$-$R^1$ (IV), un équivalent d'un ester d'acide organo-boronique de formule B-$R^1$($OR^2$)($OR^3$) (I) avec 1/n équivalent de sel $K^{n+}$ $nX^-$ (II) et 3 équivalents de métal M étant fournis dans un solvant ou un mélange de solvant S, 3 équivalents d'un composé aromatique halogéné $R^4$-Y (III) étant ajoutés, de l'eau et éventuellement un deuxième solvant organique S étant ajoutés et le composé 1/n $K^{n+}R^4{}_3B^-$-$R^1$ (IV) étant séparé avec la phase organique, et

   $R^1$ représentant un radical $C_{1-22}$-alkyle, $C_{3-22}$-alcényle, $C_{3-22}$-alcynyle, $C_{5-7}$-cycloalkyle ou $C_{7-13}$-aralkyle, éventuellement substitué par hydroxy et/ou alcoxy et/ou acyloxy et/ou halogène et
   $R^2$ et $R^3$ représentant indépendamment l'un de l'autre un radical $C_{1-22}$-alkyle éventuellement ramifié ou un radical $C_{3-7}$-cycloalkyle éventuellement substitué par alkyle ou $R^2$ et $R^3$ formant ensemble un pont carboné à 2 à 8 chaînons éventuellement substitué par alkyle et/ou interrompu par des atomes d'oxygène et
   $R^4$ représentant un radical $C_{6-10}$-aryle, qui est éventuellement substitué par au moins un radical choisi parmi halogène, $C_{1-4}$-alkyle, trifluorométhyle, $C_{1-4}$-alcoxy, trifluorométhoxy, phényle et phénoxy et
   K représentant un organocation substitué de manière quelconque de valence n à base d'azote, de phosphore, d'oxygène, de soufre et/ou d'iode et
   M représentant un métal choisi de manière quelconque parmi les métaux alcalins, le magnésium, le calcium et l'aluminium et
   X représentant un halogénure ou un alcoxyde ou un alkylsulfure et
   Y représentant iode ou brome ou chlore et
   n représentant 1, 2 ou 3 et
   S représentant un solvant organique aprotique ou un mélange de solvants aprotiques.

2. Procédé pour la préparation de composés 1/n $K^{n+}R^4{}_3B^-$-$R^1$ de formule (IV) selon la revendication 1 comprenant les étapes de

   i) mise à disposition d'un équivalent d'un ester d'acide organo-boronique B-$R^1$($OR^2$)($OR^3$) (I) avec 1/n équivalent de sel $K^{n+}$ $nX^-$ (II) et 3 équivalents de métal M dans un solvant ou un mélange de solvants S,
   ii) ajout de 3 équivalents d'un composé aromatique halogéné $R^4$-Y (III),
   iii) un réactif organométallique produit *in situ* réagissant avec les substances mises à disposition (I) et (II) pour donner 1/n $K^{n+}R^4{}_3B^-$-$R^1$ (IV) .

3. Procédé selon la revendication 1 ou 2, **caractérisé en ce que** les organocations K de valence n à base d'azote sont des ions ammonium, des ions pyridinium et des ions imidazolium, qui peuvent éventuellement porter dans une ou plusieurs chaînes latérales d'autres groupes fonctionnels comme des éthers, des esters, des amides et/ou des carbamates et qui peuvent également être présents sous forme oligomérique ou polymérique ou pontée.

4. Procédé selon la revendication 1 ou 2, **caractérisé en ce que** les organocations K de valence n à base de phosphore sont des sels de tétraalkylphosphonium, de trialkylarylphosphonium, de dialkyldiarylphosphonium, d'alkyltriarylphosphonium ou de tétraarylphosphonium, substitués d'une manière quelconque, qui peuvent éventuellement porter

dans une ou plusieurs chaînes latérales d'autres groupes fonctionnels comme des carbonyles, des amides et/ou des carbamates et qui peuvent également être présents sous forme oligomérique ou polymérique ou pontée.

5. Procédé selon la revendication 1 ou 2, **caractérisé en ce que** l'organocation K de valence n à base d'oxygène est un pyrylium substitué d'une manière quelconque, également sous forme annelée comme dans un benzopyrylium, flavylium, naphtoxanthénium ou des cations polymériques dotés des motifs de substitution mentionnés.

6. Procédé selon la revendication 1 ou 2, **caractérisé en ce que** les organocations K de valence n à base de soufre sont des composés onium du soufre, qui portent des radicaux $C_{6-12}$-alkyle, $C_{6-10}$-aryle, $C_{7-12}$-arylalkyle ou $C_{5-6}$-cycloalkyle, identiques ou différents, éventuellement substitués et/ou construisent des motifs liants répétitifs oligomériques ou polymériques pour donner des sels de sulfonium avec $1 \leq n \leq 3$ ainsi que des thiopyrylium ou des cations polymériques avec les motifs de substitution mentionnés.

7. Procédé selon la revendication 1 ou 2, **caractérisé en ce que** les organocations K de valence n à base d'iode sont des composés onium de l'iode, qui portent des radicaux $C_{4-12}$-alkyle, $C_{6-10}$-aryle, $C_{7-12}$-arylalkyle ou $C_{5-6}$-cycloalkyle, identiques ou différents, éventuellement substitués et/ou qui construisent des motifs liants répétitifs oligomériques ou polymériques pour donner des sels d'iodonium avec $1 \leq n \leq 3$, ou des cations polymériques supplémentaires avec les motifs de substitution mentionnés.

8. Procédé selon l'une quelconque des revendications 1 à 7, **caractérisé en ce que** $R^1$ représente un radical $C_{2-18}$-alkyle, $C_{3-18}$-alcényle, $C_{3-18}$-alcynyle, $C_{5-6}$-cycloalkyle, ou $C_{7-13}$-aralkyle, éventuellement substitué par hydroxy et/ou alcoxy et/ou acyloxy et/ou halogène.

9. Procédé selon l'une quelconque des revendications 1 à 8, **caractérisé en ce que** $R^2$ et $R^3$ représentent indépendamment l'un de l'autre un radical $C_{2-18}$-alkyle éventuellement ramifié ou $R^2$ et $R^3$ forment ensemble un carbocycle à 4 à 7 chaînons éventuellement substitué.

10. Procédé selon l'une quelconque des revendications 1 à 9, **caractérisé en ce que** $R^4$ représente un radical $C_{6-10}$-aryle, qui est éventuellement substitué par au moins un radical choisi parmi halogène, $C_{1-4}$-alkyle, trifluorométhyle, $C_{1-4}$-alcoxy, trifluorométhoxy, phényle et/ou phénoxy.

Figur 1:

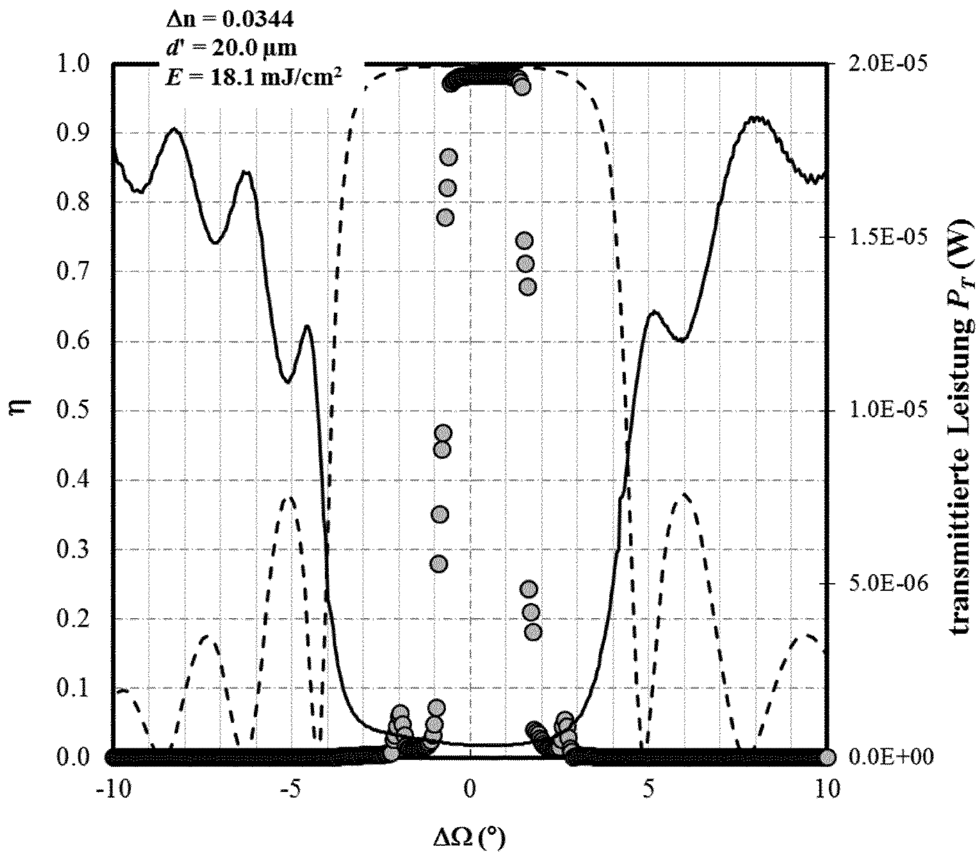
Δn = 0.0344
d' = 20.0 µm
E = 18.1 mJ/cm²
η
1.0
0.9
0.8
0.7
0.6
0.5
0.4
0.3
0.2
0.1
0.0
-10
-5
0
5
10
ΔΩ (°)
2.0E-05
1.5E-05
1.0E-05
5.0E-06
0.0E+00
transmittierte Leistung P_T (W)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19850139 A1
- JP 2002226486 A
- GB 2307474 A
- US 3125555 A
- EP 0223587 A
- WO 2012062655 A
- WO 2015091427 A
- WO 2015055576 A1
- US 5194472 A
- US 2857310 A
- DE 3723797
- US 4954635 A
- WO 2000030444 A
- US 5565290 A


### In der Beschreibung aufgeführte Nicht-Patentliteratur


- **REICHARDT, C.** Solvents and Solvent Effects in Organic Chemistry. Wiley-VCH, 2003
- *Organometallics,* 1983, vol. 2 (10), 1311-16
- *CHEMICAL ABSTRACTS,* 64-20-0, 1643-19-2, 122-18-9, 57-09-0, 121-54-0, 61546-01-8, 64697-40-1, 114569-84-5, 81505-35-3, 1224595-52-1
- *Fuel Processing Technology,* 2014, vol. 118, 296-301
- *J. Am. Chem. Soc.,* 2002, vol. 124 (11), 2604-2613
- *Zhurnal Neorganicheskoi Khimii,* 1978, vol. 23 (3), 825-6
- *Chemische Berichte,* 1984, vol. 117 (4), 1487-96
- *J. Am. Chem. Soc.,* 1955, vol. 77, 485-6
- *Taiwan Kexue,* 1959, vol. 13, 95-8
- *Chemical Engineering Science,* vol. 69 (1), 483-491
- *Chem. Ber.,* 1984, vol. 117, 1487-1496
- *Izvestiya Vysshikh Uchebnykh Zavedenii, Khimiya i Khimicheskaya Tekhnologiya,* 1973, vol. 16 (6), 891
- *Chemicky Prumysl,* 1967, vol. 17 (2), 67-9